# EUROPEAN PATENT SPECIFICATION

(11) **EP 2 484 756 B1**
(45) Date of publication and mention of the grant of the patent: **20.09.2017**
(21) Application number: 10820584.0
(22) Date of filing: 29.09.2010
(51) Int. Cl.: A61L 27/00, C07K 16/28, C07K 16/18, C12N 5/0735, C12N 5/077, C12N 5/10

(54) **NOVEL PACEMAKER CELL**
NEUARTIGE SCHRITTMACHERZELLE
NOUVELLE CELLULE GÉNÉRATRICE DE RYTHME

(30) Priority: 30.09.2009 JP 2009226760
(43) Date of publication of application: 08.08.2012
(73) Proprietor: National University Corporation Tottori University, Tottori-shi Tottori 680-8550 (JP)
(72) Inventor: HISATOME, Ichiro, Yonago-shi Tottori 683-8503 (JP); SHIRAYOSHI, Yasuaki, Yonago-shi Tottori 683-8503 (JP); MIAKE, Junichiro, Yonago-shi Tottori 683-8503 (JP)
(74) Representative: Forrest, Graham Robert
(86) International application number: PCT/JP2010/066952
(87) International publication number: WO 2011/040469

(56) References cited:
- WO-A1-2009/017254
- WO-A2-2007/014134
- WO-A2-2007/047522
- D CAPILUPO ET AL: "Acta Physiologica Congress", THE 60TH NATIONAL CONGRESS OF THE ITALIAN PHYSIOLOGICAL SOCIETY, vol. 197, 1 September 2009 (2009-09-01), XP055099679,
- MORIKAWA KUMI ET AL: "Identification, isolation and characterization of HCN4-positive pacemaking cells derived from murine embryonic stem cells during cardiac differentiation.", PACING AND CLINICAL ELECTROPHYSIOLOGY : PACE MAR 2010, vol. 33, no. 3, March 2010 (2010-03), pages 290-303, XP55099647, ISSN: 1540-8159
- OU DONG-BO ET AL: "Using embryonic stem cells to form a biological pacemaker via tissue engineering technology.", BIOESSAYS : NEWS AND REVIEWS IN MOLECULAR, CELLULAR AND DEVELOPMENTAL BIOLOGY FEB 2009, vol. 31, no. 2, February 2009 (2009-02), pages 246-252, XP55099695, ISSN: 1521-1878
- CHUNG YOUNG ET AL: "Human embryonic stem cell lines generated without embryo destruction", CELL STEM CELL, vol. 2, no. 2, February 2008 (2008-02), pages 113-117, XP002719579, ISSN: 1934-5909
- YASUAKI SHIRAYOSHI ET AL.: 'Ion Channel o Kashika Senbetsu Marker to shita ES Saibo Yurai Seibutsugakuteki Pacemaker Saibo no Juritsu to sono Tokusei no Kento' SHINDENZU vol. 28, no. 5, 2008, page 466, XP008157434
- YANO, S. ET AL.: 'Changes of HCN gene expression and If currents in Nkx2.5-positive cardiomyocytes derived from murine embryonic stem cells during differentiation' BIOMED RES vol. 29, no. 4, 2008, pages 195 - 203, XP008154655
- KENTOKU YANAGI ET AL.: 'ES Saibo Yurai Shinkin Pacemaker Saibo no Jidono Iji ni Okeru Ion Channel HCN1, 4 Oyobi Cav3.1 no Igi' FOLIA ENDOCRINOLOGICA JAPONICA vol. 82, no. 2, 2006, page 512, XP008154720
- BARBUTI, A. ET AL.: 'Molecular composition and Functional properties of f-channels in murine embryonic stem cell-derived pacemaker cells' JOURNAL OF MOLECULAR AND CELLULAR CARDIOLOGY vol. 46, no. 3, March 2009, pages 343 - 351, XP008154569
- YANAGI, K. ET AL.: 'Hyperpolarization-activated cyclic nucleotide-gated channels and T-type calcium channels confer automaticity of embryonic stem cell-derived cardiomyocytes' STEM CELLS vol. 25, no. 11, 2007, DAYTON, OHIO, pages 2712 - 2719, XP008154663
- YANG X.J. ET AL.: 'Mesenchymal stem cells as a gene delivery system to create biological pacemaker cells in vitro' THE JOURNAL OF INTERNATIONAL MEDICAL RESEARCH vol. 36, no. 5, 2008, pages 1049 - 1055, XP008154681
- BOINK, G.J. ET AL.: 'Engineering physiologically controlled pacemaker cells with lentiviral HCN4 gene transfer' THE JOURNAL OF GENE MEDICINE vol. 10, no. 5, 2008, pages 487 - 497, XP008154664
- SHIRAYOSHI, Y. ET AL.: 'Identification, Isolation and Characterization of HCN4- positive Cardiac Pacemaking Cells Derived from Murine EGFP Knock-in Embryonic Stem Cells at HCN4 Locus' CIRC J vol. 74, no. 1, 01 March 2010, page 400, XP008155157
- SHIN'ICHI ITO ET AL.: 'ES Saibo Yurai Shinzo Pacemaker Saibo no Bunshu to Tokusei Kaiseki' SAISEI IRYO vol. 9, 05 February 2010, page 216, XP008158415

### SEQUENCE LISTING

<110> NATIONAL UNIVERSITY CORPORATION TOTTORI UNIVERSITY
<120> NOVEL PACEMAKER CELLS
<130> 669871
<150> JP2009-226760
   <151> 2009-09-30
<160> 46
<210> 1
   <211> 11
   <212> PRT
   <213> mouse
<400> 1
   Val Ser Ile Asn Gly Met Val Asn Asn Ser Trp
   1               5                   10
<210> 2
   <211> 10
   <212> PRT
   <213> mouse
<400> 2
   Val Pro Met Leu Gln Asp Phe Pro His Asp
   1               5                   10
<210> 3
   <211> 12
   <212> PRT
   <213> Artificial Sequence
<220>
   <223> Amino acid sequence used for producing HCN4-specific antobody
<400> 3
   Val Ser Ile Asn Gly Met Val Asn Asn Ser Trp Cys
   1               5                   10
<210> 4
   <211> 11
   <212> PRT
   <213> Artificial Sequence
<220>
   <223> Amino acid sequence used for producing HCN4-specific antobody
<400> 4
   Cys Val Pro Met Leu Gln Asp Phe Pro His Asp
   1               5                   10
<210> 5
   <211> 21
   <212> DNA
   <213> Artificial Sequence
<220>
   <223> Nucleotide sequence used as a primer in PCR reaction
<400> 5
   tgaacgcgaa gctcacactg g 21
<210> 6
   <211> 20
   <212> DNA
   <213> Artificial Sequence
<220>
   <223> Nucleotide sequence used as a primer in PCR reaction
<400> 6
   tccaccaccc tgttgctgta 20
<210> 7
   <211> 20
   <212> DNA
   <213> Artificial Sequence
<220>
   <223> Nucleotide sequence used as a primer in PCR reaction
<400> 7
   agatcactca catcgccaat 20
<210> 8
   <211> 20
   <212> DNA
   <213> Artificial Sequence
<220>
   <223> Nucleotide sequence used as a primer in PCR reaction
<400> 8
   aaggtgtcct gtagcctcat 20
<210> 9
   <211> 18
   <212> DNA
   <213> Artificial Sequence
<220>
   <223> Nucleotide sequence used as a primer in PCR reaction
<400> 9
   gcaagaactc tcctccat 18
<210> 10
   <211> 18
   <212> DNA
   <213> Artificial Sequence
<220>
   <223> Nucleotide sequence used as a primer in PCR reaction
<400> 10
   atactccact ggtgctga 18
<210> 11
   <211> 20
   <212> DNA
   <213> Artificial Sequence
<220>
   <223> Nucleotide sequence used as a primer in PCR reaction
<400> 11
   cattacacac cactgacgca 20
<210> 12
   <211> 20
   <212> DNA
   <213> Artificial Sequence
<220>
   <223> Nucleotide sequence used as a primer in PCR reaction
<400> 12
   catagatggg ggtgacacag 20
<210> 13
   <211> 20
   <212> DNA
   <213> Artificial Sequence
<220>
   <223> Nucleotide sequence used as a primer in PCR reaction
<400> 13
   ctgcggcctc tacatgaagc 20
<210> 14
   <211> 20
   <212> DNA
   <213> Artificial Sequence
<220>
   <223> Nucleotide sequence used as a primer in PCR reaction
<400> 14
   tcttcactgc tgctgctgct 20
<210> 15
   <211> 20
   <212> DNA
   <213> Artificial Sequence
<220>
   <223> Nucleotide sequence used as a primer in PCR reaction
<400> 15
   caagtgctct cctgctttcc 20
<210> 16
   <211> 19
   <212> DNA
   <213> Artificial Sequence
<220>
   <223> Nucleotide sequence used as a primer in PCR reaction
<400> 16
   ggctttgtcc agctccact 19
<210> 17
   <211> 20
   <212> DNA
   <213> Artificial Sequence
<220>
   <223> Nucleotide sequence used as a primer in PCR reaction
<400> 17
   cccttcgaga tacccacaac 20
<210> 18
   <211> 20
   <212> DNA
   <213> Artificial Sequence
<220>
   <223> Nucleotide sequence used as a primer in PCR reaction
<400> 19
   tgcccatcct tcagagagtc 20
<210> 18
   <211> 20
   <212> DNA
   <213> Artificial Sequence
<220>
   <223> Nucleotide sequence used as a primer in PCR reaction
<400> 19
   atgacagcca gacagacagt 20
<210> 20
   <211> 20
   <212> DNA
   <213> Artificial Sequence
<220>
   <223> Nucleotide sequence used as a primer in PCR reaction
<400> 20
   ggtgtctgtg tcatctgagt 20
<210> 21
   <211> 18
   <212> DNA
   <213> Artificial Sequence
<220>
   <223> Nucleotide sequence used as a primer in PCR reaction
<400> 21
   ctgcagccga cagctcat 18
<210> 22
   <211> 20
   <212> DNA
   <213> Artificial Sequence
<220>
   <223> Nucleotide sequence used as a primer in PCR reaction
<400> 22
   ctgcctagcc gagatgggtt 20
<210> 23
   <211> 20
   <212> DNA
   <213> Artificial Sequence
<220>
   <223> Nucleotide sequence used as a primer in PCR reaction
<400> 23
   gagatggtca tcacgaagtc 20
<210> 24
   <211> 20
   <212> DNA
   <213> Artificial Sequence
<220>
   <223> Nucleotide sequence used as a primer in PCR reaction
<400> 24
   ggaaggccaa agtaaatccg 20
<210> 25
   <211> 18
   <212> DNA
   <213> Artificial Sequence
<220>
   <223> Nucleotide sequence used as a primer in PCR reaction
<400> 25
   cagaatcgtg ggacccat 18
<210> 26
   <211> 18
   <212> DNA
   <213> Artificial Sequence
<220>
   <223> Nucleotide sequence used as a primer in PCR reaction
<400> 26
   cggcgtccag gtttctag 18
<210> 27
   <211> 18
   <212> DNA
   <213> Artificial Sequence
<220>
   <223> Nucleotide sequence used as a primer in PCR reaction
<400> 27
   cagaatcgtg ggacccat 18
<210> 28
   <211> 18
   <212> DNA
   <213> Artificial Sequence
<220>
   <223> Nucleotide sequence used as a primer in PCR reaction
<400> 28
   cggcgtccag gtttctag 18
<210> 29
   <211> 20
   <212> DNA
   <213> Artificial Sequence
<220>
   <223> Nucleotide sequence used as a primer in PCR reaction
<400> 29
   aaggtgtttg atcccgaggg 20
<210> 30
   <211> 20
   <212> DNA
   <213> Artificial Sequence
<220>
   <223> Nucleotide sequence used as a primer in PCR reaction
<400> 30
   gggaaaggct gcgaacatct 20
<210> 31
   <211> 20
   <212> DNA
   <213> Artificial Sequence
<220>
   <223> Nucleotide sequence used as a primer in PCR reaction
<400> 31
   tgacccaggc agacaagttc 20
<210> 32
   <211> 20
   <212> DNA
   <213> Artificial Sequence
<220>
   <223> Nucleotide sequence used as a primer in PCR reaction
<400> 32
   cgtgggtgat gatgtagcag 20
<210> 33
   <211> 20
   <212> DNA
   <213> Artificial Sequence
<220>
   <223> Nucleotide sequence used as a primer in PCR reaction
<400> 33
   ctccactttg atctccagac 20
<210> 34
   <211> 20
   <212> DNA
   <213> Artificial Sequence
<220>
   <223> Nucleotide sequence used as a primer in PCR reaction
<400> 34
   ttctgcatct gggtctgtat 20
<210> 35
   <211> 20
   <212> DNA
   <213> Artificial Sequence
<220>
   <223> Nucleotide sequence used as a primer in PCR reaction
<400> 35
   gacaatttca acgaggtgct 20
<210> 36
   <211> 20
   <212> DNA
   <213> Artificial Sequence
<220>
   <223> Nucleotide sequence used as a primer in PCR reaction
<400> 36
   ccatctcacg gtcatatttg 20
<210> 37
   <211> 20
   <212> DNA
   <213> Artificial Sequence
<220>
   <223> Nucleotide sequence used as a primer in PCR reaction
<400> 37
   aaccctccat gccagcctat 20
<210> 38
   <211> 20
   <212> DNA
   <213> Artificial Sequence
<220>
   <223> Nucleotide sequence used as a primer in PCR reaction
<400> 38
   cttccagagc ctttacgcct 20
<210> 39
   <211> 20
   <212> DNA
   <213> Artificial Sequence
<220>
   <223> Nucleotide sequence used as a primer in PCR reaction
<400> 39
   cgacagcgca tccatgacta 20
<210> 40
   <211> 20
   <212> DNA
   <213> Artificial Sequence
<220>
   <223> Nucleotide sequence used as a primer in PCR reaction
<400> 40
   gctggaagac ctcgaaacgc 20
<210> 41
   <211> 20
   <212> DNA
   <213> Artificial Sequence
<220>
   <223> Nucleotide sequence used as a primer in PCR reaction
<400> 41
   gctgtttggg aggctagaat 20
<210> 42
   <211> 20
   <212> DNA
   <213> Artificial Sequence
<220>
   <223> Nucleotide sequence used as a primer in PCR reaction
<400> 42
   cgaaggtgac gaagtagacg 20
<210> 43
   <211> 24
   <212> DNA
   <213> Artificial Sequence
<220>
   <223> Nucleotide sequence used as a primer in PCR reaction
<400> 43
   ctcagctcta aggcccaggt cccg 24
<210> 44
   <211> 21
   <212> DNA
   <213> Artificial Sequence
<220>
   <223> Nucleotide sequence used as a primer in PCR reaction
<400> 44
   ccgcgctgcg atggcaaaga g 21
<210> 45
   <211> 20
   <212> DNA
   <213> Artificial Sequence
<220>
   <223> Nucleotide sequence used as a primer in PCR reaction
<400> 45
   ctcctccccc tgacttcaat 20
<210> 46
   <211> 20
   <212> DNA
   <213> Artificial Sequence
<220>
   <223> Nucleotide sequence used as a primer in PCR reaction
<400> 46
   cgccgtttgt cactatggta 20

## Description

### [Technical Field]

The present disclosure relates to a cardiac pacemaker cell, particularly a pacemaker cell, the beating rate of which can be controlled, a method for obtaining the same, use thereof, and the like.

### [Background Art]

Bradyarrhythmia, which significantly decreases the beating rate, is a cardiac arrhythmia of which the incidence increases with age and which causes sudden death, heart failure, and cerebral stroke, and has significant social influences in Japan, which has a declining birthrate and an aging population. Therapeutic treatments for the disease have no effective procedure other than implantation of an artificial pacemaker which is employed to increase the beating rate and works by battery, and such artificial pacemakers are implanted in about fifty thousand patients each year. Implantation of an artificial pacemaker (1) needs surgical operations and thus is highly invasive to the body, and (2) is followed by requiring re-implantation every five to eight years due to the battery being consumed. In addition, (3) the beating rate does not increase in concert with the degree of activities of the body, thereby leading to an inevitable decrease in quality of life (QOL). It is known that embryonic stem (ES) cells and induced pluripotent stem cells are able to differentiate *in vitro* into three germ layers, and particularly when aggregates of ES cells (embryoid bodies) are formed, to differentiate into cardiac muscle cells (cardiomyocytes), among which pacemaker cells are included (Non-Patent Literature 1).

Biological pacemakers made from cells can generate electrical signals by themselves, and can make up for the above-mentioned disadvantages of artificial pacemakers, if these biological pacemakers can be under control of the autonomic nerve. In order to make biological pacemakers from cells, it is necessary to select and collect pacemaker cells which are generated during cardiac differentiation from stem cells. As described above, embryonic stem cells and induced pluripotent stem cells are capable of inducing cardiac differentiation with relative ease by subjecting them to three-dimensional culture. Selective collection of only pacemaker cells, however, has not been achieved yet. A main reason for this is that there are not found markers allowing one to select pacemaker cells.

Capilupo et al., Acta Physiologica Congress, 197 (2009) describes the isolation and characterisation of cardiac pacemaker myocytes using HCN4 as a marker.

Morikawa et al., PACE, 33: 290-303 (2010) describes the isolation and characterisation of HCN4-expressing pacemaking cells derived from murine embryonic stem cells.

Ou et al., Bioessays, 31: 246-252 describes gene-based and cell-based approaches to producing biological pacemakers.

### [Citation List]

### [Non-Patent Literature]

### [Non-Patent Literature 1]

Yano S, Miake J, Mizuta E, Manabe K, Bahrudin U, Morikawa K, Arakawa K, et al., Changes of HCN gene expression and I(f) currents in Nkx2.5-positive cardiomyocytes derived from murine embryonic stem cells during differentiation. Biomed Res 2008; 29:195-203

### [Summary]

### [Technical Problem]

Although studies, whether in Japan or in others, have been made in which heart-specific transcription factors are used to select and collect cardiomyocytes of various types, none of them are successful in selecting and collecting pacemaker cells. Furthermore, when applications to future regenerative medicine are considered, it is necessary to establish a method for selection for only pacemaker cells without the need for genetic modification of embryonic stem cells or induced pluripotent stem cells. In addition, if it is possible to obtain pacemaker cells, the beating rate of which is controlled as needed and at will, then such pacemaker cells will allow avoiding patients' decreased QOL due to the beating rate which does not increase in concert with the degree of activities of the body.

The present inventors assumed that ion channels which are specifically present on the cell surface of pacemaker cells would be able to serve as markers to select pacemaker cells. If this be possible, the use of antibodies reacting to these ion channels will allow one to collect pacemaker cells and facilitate their application to regenerative medicine, without genetic modification of embryonic stem cells or induced pluripotent stem cells.

### [Solution to Problem]

In order to solve the problems as mentioned above, the present inventors have intensively studied on the basis of the above-described assumption and conducted an extensive genetic analysis and found that channels specific for pacemaker cells are expressed during cardiac differentiation of ES cells; that when system in which an HCN-GFP reporter gene has been knocked-in in mouse ES cells are used, HCN4 channel is a marker useful for selecting and collecting pacemaker cells; and that the cells obtained in this approach are provided with characteristics as pacemaker cell of exhibiting expression of HCN, Ca, and HERG channels, without expression of inward-rectifier K channels. On the other hand, contrary to the inventors' expectations, the present inventors found that the pacemaker cells according to the present disclosure possess Na channels and additionally, are novel pacemaker cells, the beating rate of which can be controlled by control of Na channel. Furthermore, the present inventors have found that when the pacemaker cells are implanted to cardiac muscle of animals using cell aggregates which are formed by assembling the pacemaker cells, their strong and permanent functions as a pacemaker are exerted and the beating rate of their hearts is also controllable. In addition, the present inventors were successful in the generation of antibodies capable of recognizing these pacemaker cells.

Accordingly, the following is herein disclosed:
(i) it is possible to induce differentiation of ES cells into cardiac muscle, thereby to collect selectively pacemaker cells using HCN4 channel as marker;
(ii) the pacemaker cells according to the present disclosure are ones which express many Na channels, in addition to having fundamental properties as pacemaker cells, the beating rate of which can be controlled by control of Na channel;
(iii) the present disclosure can be applied also to human ES and iPS cells;
(iv) the pacemaker cells disclosed herein exert strong and permanent functions as a pacemaker when they are actually implanted to the heart as a cell aggregate(s) which is/are formed by assembling more than a certain number of the pacemaker cells, and in addition, the beating rate is controllable, for example, via regulation of the autonomic nerve; and
(v) an antibody capable of recognizing the pacemaker cells disclosed herein can be produced using a particular amino acid sequence of an extracellular domain of HCN4 as the antigen recognition site.

The invention is as defined in the claims. The following is also described herein:
(1) a pacemaker cell which possesses HCN4 channel and Na channel, the beating rate of which can be controlled by control of Na channel, wherein the cell is derived from embryonic stem (ES) cells, or induced pluripotent stem (iPS) cells;
(2) a method for obtaining a pacemaker cell, the beating rate of which can be controled by regulation of Na channel, which comprises inducing differentiation of embryonic stem (ES) cells, or induced pluripotent stem (iPS) cells into cardiac muscle, and selecting for the pacemaker cell using HCN4 channel as marker;
(3) a method for the producing a pacemaker cell, the beating rate of which can be controlled by regulation of Na channel, which comprises introducing HCN4 channel gene into embryonic stem (ES) cells, or induced pluripotent stem (iPS) cells, subjecting to differentiation induction into cardiac muscle, and selecting and obtaining cells positive for a label;
(4) the method for the producing a pacemaker cell according to (3), which comprises incorporating a gene encoding a selectable label into HCN4 channel gene, to construct a targeting gene, and introducing the targeting gene into embryonic stem (ES) cells, or induced pluripotent stem (iPS) cells;
(5) a pacemaker cell which is obtainable by the method according to any one of (2) to (4), wherein the cell possesses HCN channel and Na channel, the beating rate of which can be controlled by regulation of Na channel;
(6) an implant comprising the pacemaker cell according to (1) or (5);
(7) the implant according to (6), wherein the pacemaker cell is in the form of a cell aggregate formed therefrom;
(8) the implant according to (7), wherein the cell aggregate comprises the pacemaker cell in a number of about 60,000 cells or more;
(9) the implant according to (7), wherein the cell aggregate comprises the pacemaker cell in a number of about 90,000 cells or more;
(10) a cardiac pacemaker comprising, as the essential component, the pacemaker cell according to (1) or (5), or the implant according to any one of (6) to (9), the beating rate of which can be controlled by regulation of Na channel;
(11) the pacemaker cell according to (1) or (5), or the implant according to any one of (6) to (9), for use in the production of a cardiac pacemaker, the beating rate of which can be controlled by regulation of Na channel;
(12) a method for the producing a cardiac pacemaker, the beating rate of which can be controlled by regulation of Na channel, which comprises using the pacemaker cell according to (1) or (5), or the implant according to any one of (6) to (9);
(13) an HCN4 specific antibody, which is obtainable by using an amino acid sequence VSINGMVNNSW (SEQ ID NO:1) or VPMLQDFPHD (SEQ ID NO:2) as the antigen and recognizes the amino acid sequence as the recognition site; and
(14) the method according to any one of (2) to (4), which comprises selecting or selection for the pacemaker cell using the antibody according to (13).

### [Advantageous Effects]

According to the present disclosure, there is provided a pacemaker cell, the beating rate of which can be controlled. The beating rate of the pacemaker cell can be controlled as needed and at will, thereby resulting in the prevention of patients' decreased QOL. Further, the method for obtaining a pacemaker cell according to the present disclosure enables to obtain only pacemaker cells without the need for genetic modification of embryonic stem (ES) cells or induced pluripotent stem (iPS) cells, and thus is also suitable for applications to regenerative medicine. In addition, the pacemaker cells described herein exert strong and long-lasting functions as a pacemaker when they are implanted to cardiac muscle as a cell aggregate(s) which is/are formed by assembling a certain number or more of the pacemaker cells.

### [Brief Description of Drawings]

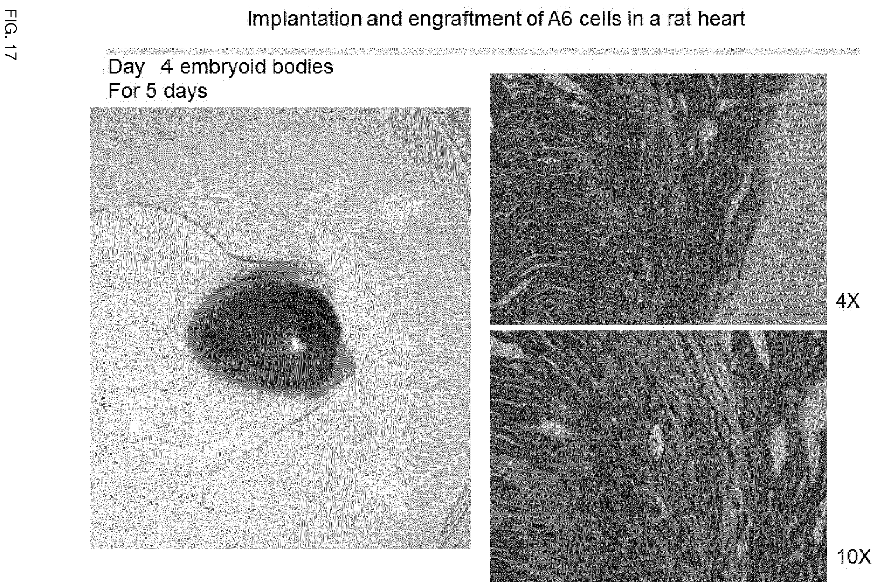

Fig. 1 is a schematic representation of a targeting gene in which a selection/collection label, a GFP protein gene, is connected into the promoter region of HCN4 channel gene. The targeting gene was used for homologous gene recombination of the GFP gene into the HCN4 locus in an AB1 (wild-type) ES cell strain, establishing knocked-in ES cells.
Fig. 2 shows results of selection of HCN4-GFP gene knocked-in ES cells. The establishment of HCN4-GFP gene knocked-in ES cells was verified by PCR and southern blot methods.
Fig. 3 is a graph showing changes in the beating rate of embryoid bodies during the differentiation induction of HCN4-GFP gene knocked-in ES cells to a heart.
Fig. 4 indicates observation results using an incubation imaging system, showing a relationship between beating sites and GFP expression in H7 embryoid body. The sites of beating and of GFP expression are indicated by the arrows and surrounded by white broken lines, respectively.
Fig. 5 indicates a result showing that only cells positive for GFP signal were selected and collected using FACS.
Fig. 6 indicates microscopic graphs showing the morphology of HCN4-GFP positive cells derived from H7 embryoid body.
Fig. 7 indicates the numbers of beating cells at days 11, 13, and 14 during the differentiation of GFP-signal positive cells.
Fig. 8 shows the results from examining the expression of cardiac pacemaker genes in HCN4-GFP positive cells derived from H7 embryoid body at day 11 after differentiation induction. "+" represents a GFP positive fraction, and "-" represents a GFP negative fraction.
Fig. 9 shows the results from immunostaining of HCN4-GFP positive cells derived from H7 embryoid body at days 7, 10, and 15 after differentiation induction. "7+" and "7-," "10+" and "10-," and "15+" and "15-" represent a GFP positive fraction and a GFP negative fraction at days 7, 10, and 15 after differentiation induction, respectively.
Fig. 10 shows the results from immunostaining of HCN4-GFP positive cells derived from H7 embryoid body at day 18 after differentiation induction.
Fig. 11 shows the results from the analysis of the automaticity driven by HCN4 channel in HCN4-GFP positive cells derived from H7 embryoid body.
Fig. 12 shows the results from the analysis of the ion channels responsible for pacemaker activities of HCN4-GFP positive cells derived from H7 embryoid body.
Fig. 13 shows the results from simulating the electrical activity of GFP-positive cells when Na channel was suppressed.
Fig. 14 is a graph showing a relationship between concentration of lidocaine, which is Na channel inhibitor, and the beating rate of pacemaker cells.
Fig. 15 shows a positive chronotropic effect of HCN4-GFP positive cells derived from H7 embryoid body via β-1 receptor stimulation. Particularly, Fig. 15 represents figures showing that the beating rate of the pacemaker cells is increased by means of isoproterenol, which is an autonomic stimulant. The HCN4-GFP positive cells are pacemaker cells which are controlled by the sympathetic nerve.
Fig. 16 indicates graphs showing that the beating rate of the pacemaker cells is increased in response to isoproterenol, which is a sympathetic stimulant, and decreased in response to carbachol, which is a parasympathetic stimulant.
The left panel in Fig. 17 indicates a picture of X-gal staining of a heart which was subjected to implantation of a cell aggregate comprising organized pacemaker cells. Each of the right panels in Fig. 17 indicates microscopic views from X-gal staining of cardiac muscle sections of the heart shown in the left panel.

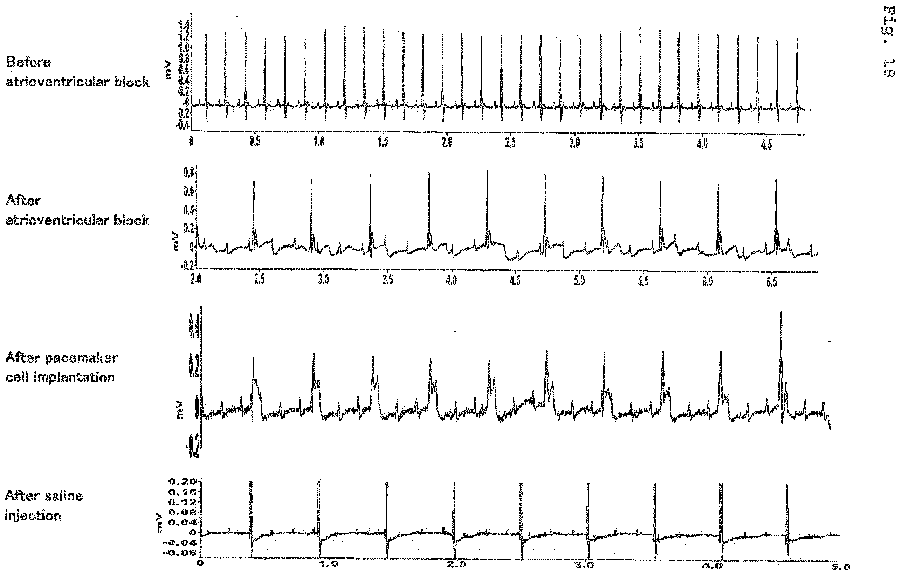

Fig. 18 indicates electrocardiograms showing effects of implanting the pacemaker cells to the heart of a rat with atrioventricular block. The abscissa axis represents time (in seconds) and the ordinate axis represents electrical potentials (in mV).

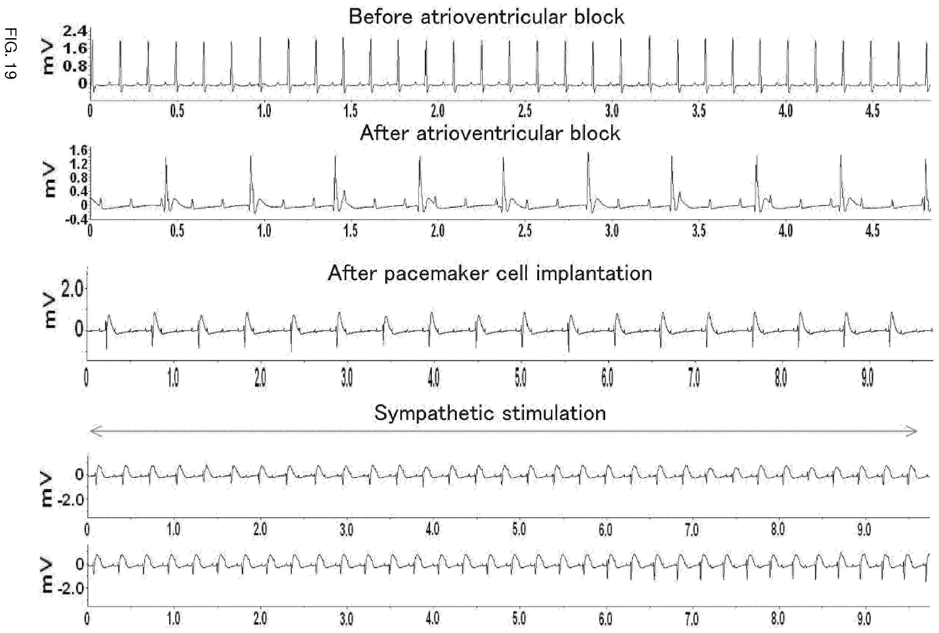

Fig. 19 indicates electrocardiograms showing effects of implanting the pacemaker cells to the heart of a rat with atrioventricular block and showing effects of intraperitoneal administration of isoproterenol, which is a sympathetic stimulant, on the beating rate. The abscissa axis represents time (in seconds) and the ordinate axis represents electrical potential (in mV).

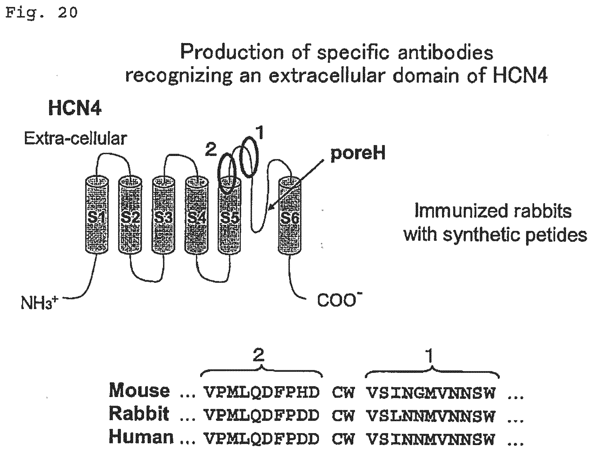

Fig. 20 is a schematic representation of procedures for producing specific antibodies recognizing an extracellular domain of HCN4.

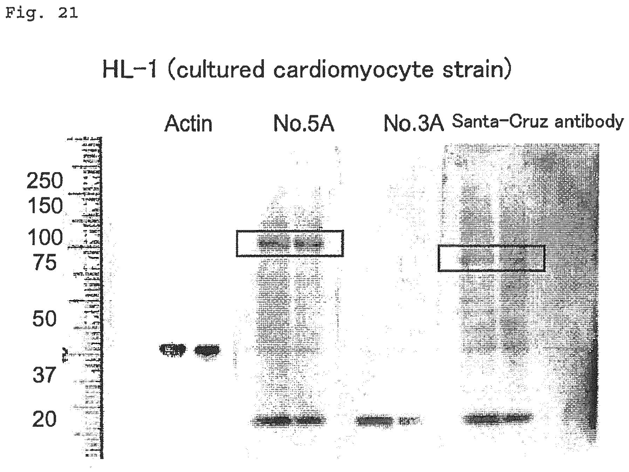

Fig. 21 indicates a western blot showing that a specific antibody recognizing an extracellular domain of HCN4 recognizes HCN channel of HL-1 cells. A specific antibody which recognizes an HCN4 extracellular domain recognizes HCN channel of HL-1 cells.

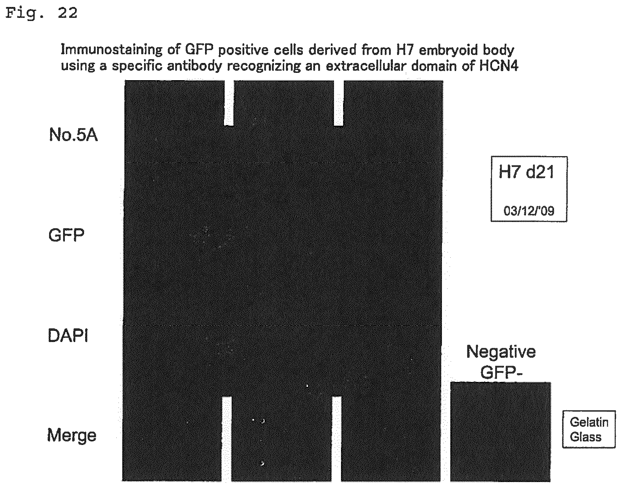

Fig. 22 indicates an immunostaining view of HCN4-GFP positive cells derived from H7 embryoid body using a specific antibody recognizing an extracellular domain of HCN4.

### [Description of Embodiments of the disclosure]

The present disclosure is primarily based on the following findings.
(I) Studies using a computer simulation model for electrical characteristics of heart pacemaker cells responsible for heart beating reveal that in characteristic ion channels having a key role in properties of the pacemaker cells described herein, outward channels, such as the HCN gene family, Ca channel gene family, and HERG channels, are important. The currents generated by these channels were found to be able to result in the automaticity of the pacemaker cells.
(II) Extensive gene analysis found that a channel characteristic of pacemaker cells (HCN channel) is expressed during the cardiac differentiation of ES cells and in addition, HCN4 channel is expressed in ES cell-derived cardiomyocytes.
(III) A reporter gene was constructed in which a selection/collection label, a GFP protein gene, was joined into the promoter region of HCN4 channel gene, and subjected to gene introduction, thereby to establish cells having the reporter gene incorporated into an allele on one chromosome. Using the fluorescence of GFP from the established ES cells as a marker resulted in successful selection and collection of the pacemaker cells according to the present disclosure.
(IV) It was ascertained that among the selected and collected pacemaker cells there are cells exhibiting automaticity, which possess properties common to pacemaker cells and are controlled by the autonomic nerve. On the other hand, it was proved in cell-biological and electrophysiological experiments that the pacemaker cells express Na channels and in pharmacological experiments that their beating rate can be controlled under regulation of the Na channel. Based on these findings, a model was made in which factors of Na channels were incorporated into the computer simulation model for pacemaker cells, and reproduced the experimental results.
(V) It was ascertained, from experiments in which cell aggregates were implanted to the heart muscle of animals after atrioventricular block, that the pacemaker cells described herein can exert strong and permanent functions as a pacemaker when a certain number or more of the pacemaker cells are assembled to form the cell aggregates. In addition, the beating rate of the heart to which the pacemaker cells were implanted was able to be controlled through excitation of the autonomic nerve.
(VI) An antibody capable of recognizing the inventive cells could be produced using a particular amino acid sequence of an extracellular domain of HCN4 as the antigen recognition site, and it was verified that the antibody was be able to recognize the pacemaker cells according to the present disclosure.

Therefore, the present disclosure, in one aspect, provides a pacemaker cell which possesses HCN channel and Na channel, the beating rate of which can be controlled by regulation of the Na channel, wherein the cell is derived from ES cells or iPS cells. The HCN channel of the pacemaker cell may be any HCN channel of HCN1, HCN2, HCN3, and HCN4, and preferably is HCN4 channel. The species of animals from which the ES or iPS cells are derived may be any species. It is preferable that the ES or iPS cells are ones which are derived from the same species as that of an animal to be treated with the pacemaker cell.

The pacemaker cells according to present disclosure are characterized in that the beating rate (or heart beat) can be controlled as needed and at will, by inhibiting or activating Na channel. In order to increase the beating rate of the pacemaker cells, Na channel activators may be allowed to act on these cells. Particularly, Na channel activators which can be used include salicylates, benzoates, and the like. In order to decrease the beating rate of the pacemaker cells, on the other hand, Na channel inhibitors may be allowed to act on these cells. Particularly, Na channel inhibitors which can be used include lidocaine, plisicainide, procainamide, and the like. In addition, the pacemaker cells disclosed herein can increase the beating rate (of the heart) by means of sympathetic stimulants. Sympathetic stimulants which can be used include isoproterenol, norepinephrine, and the like. Also, the pacemaker cells can decrease the beating rate (of the heart) by means of parasympathetic stimulants. Parasympathetic stimulants which can be used include carbachol, acetylcholine, and the like. These drugs are exemplary and not intended to be limiting.

In another aspect, the present disclosure provides a method for obtaining a pacemaker cell, the beating rate of which can be controlled by regulation of the Na channel, said method comprising inducing differentiation of ES cells, or iPS cells, into cardiac muscle, and selecting for the pacemaker cell using HCN4 channel as a marker. In conventional selection of pacemaker cells, proteins which are present within the cells are used as selection markers, and there are no selection procedures in which ion channels are used as selection markers. Now, it has turned out for the first time that HCN channel can be used as a marker to obtain pacemaker cells. It is HCN channel that can be used as a marker in the method for obtaining a pacemaker cell described herein. Any of HCN1, HCN2, HCN3 and HCN4 channels may be used, with HCN4 channel being preferable.

In the above-described method for obtaining a pacemaker cell, preferable starting materials which can be used are embryonic stem (ES) cells, or induced pluripotent stem (iPS) cells. Procedures and methods for inducing differentiation of embryonic stem (ES) cells, or induced pluripotent stem (iPS) cells, into cardiac muscle can appropriately be selected and employed by those skilled in the art, and medium components and culture conditions for these purposes can also be employed from among those known in the art. Detection of HCN channel on cells which have been induced and differentiated into cardiac muscle can be performed by known methods, such as methods using antibodies specific for HCN (preferably HCN4) channel. Methods for such detection are not limited in particular. Preferably, it is preferred that an antibody to be used or HCN channel to be expressed is labeled with a detectable label. Particularly preferably, the pacemaker cells are obtained using HCN (preferably HCN4) channel as a marker, using FACS with a cell sorter.

The present disclosure, in a further aspect, provides a method for the producing a pacemaker cell, the beating rate of which can be controlled by regulation of the Na channel activities, said method comprising introducing HCN (preferably HCN4) channel gene into embryonic stem (ES) cells, or induced pluripotent stem (iPS) cells, subjecting to differentiation induction into cardiac muscle, and selection for and obtaining cells positive for a label. Procedures and methods for introducing HCN channel gene into the above-mentioned cells are well known to those skilled in the art and examples of such procedures and methods include, but are not limited to, those using CaCl₂, lipofectamine, nucloefector, electroporation, and the like.

In the above-described method for the producing a pacemaker cell, it is also possible that a selectable label is attached to HCN (preferably HCN4) channel gene, which then is introduced into the above-mentioned cells. Use of such an approach will make it easier to select and collect cells having HCN channel. As shown in the Example, a targeting gene may be constructed by incorporating a gene encoding a selectable label (for example, a GFP gene, an RFP gene, or other genes that encode proteins or peptides capable of external detection or binding) into HCN (preferably HCN4) channel gene and introduced into embryonic stem (ES) cells (instead of which, induced pluripotent stem (iPS) cells, can be used), which are then subjected to differentiation induction into cardiac muscle, followed by selection for and obtaining cells positive for the label, that is, cells which possess HCN channel, thereby obtaining the pacemaker cells according to the present disclosure. It is preferable to incorporate a gene encoding a selectable label into the promoter region of the HCN gene. Such a label is preferably GFP, RFP, binding sites of an antibody, for example, recognition sites of antibodies directed against HCN4, such as SEQ ID NO:1 or 2, and the like.

Pacemaker cells which were selected and obtained using HCN4 channel as a marker were found out to be provided with characteristics as pacemaker cells of exhibiting expression of Ca and HERG channels, besides HCN4 channel and Na channels, without expression of inward-rectifier K channels. It was unexpected, on the other hand, that the pacemaker cells collected as described above possess Na channels, and further these pacemaker cells were novel type cells, the beating rate of which can be controlled by regulation of the Na channel.

Also described herein is a pacemaker cell which is obtained by the collecting method as described above, wherein the cell possesses HCN channel and Na channel, the beating rate of which can be controlled by regulation of the Na channel.

The pacemaker cells according to the present disclosure can be used to treat or ameliorate arrhythmias. The type of arrhythmias which can be treated or ameliorated is that which can be treated or ameliorated with conventional pacemakers, and includes, but is not limited to, for example, bradyarrhythmias such as bradycardic ventricular fibrillation, atrioventricular block, sick sinus syndrome, and the like. Suspensions of the pacemaker cells according to the present disclosure may be applied directly to the heart. In this case, the pacemaker cells may be contained in appropriate carriers or substrates to form implants. Carriers or substrates which can be used include temperature-sensitive culture dishes, fibrin glues, and the like, with temperature-sensitive culture dishes being preferable. Also preferable are biodegradable carriers or substrates, like biodegradable cellulose, which are decomposed after the pacemaker cells have been settled on the heart and exert functions. In order to make the pacemaker cells contained in carriers or substrates, the pacemaker cells may be attached or immobilized on the surface of carriers or substrates, or alternatively embedded in carriers or substrates. The pacemaker cells may also be contained in sheet-typed carriers or substrates made of temperature-sensitive culture dishes, or alternatively in matrices such as matrigel, followed by their application to the heart.

More preferably, the pacemaker cells according to the present disclosure may be employed in pacemakers, as cell aggregates which are formed by assembling the pacemaker cells. The pacemaker cells can exert strong and permanent functions as a pacemaker by organizing them as a cell aggregate. A cell aggregate which is obtained by assembling the pacemaker cells comprises the pacemaker cells, and may comprise cells of types other than the pacemaker cells, substances for exerting functions of the pacemaker cells, and the like. The cell aggregate comprises about 60,000 or more, about 70,000 or more, or about 80,000 or more, preferably about 90,000 or more, more preferably about 150,000 or more, and still more preferably about 200,000 or more, of the pacemaker cells. The above-described numbers of the pacemaker cells may be used as a single cell-aggregate or as a plurality of cell-aggregates (for example, two to several, 10 to 20, etc.). For example, 30 cell-aggregates, each of which comprises about 3,000 cells of the pacemaker cells, may be implanted to the heart. For example, 5 cell-aggregates, each of which comprises about 3,000 cells of the pacemaker cells, may be gathered to make a larger cell-aggregate, and then four of these larger cell-aggregates are implanted to the heart. For example, 5 cell-aggregates, each of which comprises about 3,000 cells of the pacemaker cells, may be gathered to make a larger cell-aggregate, and then six of these larger cell-aggregates are implanted to the heart. The shapes of the cell aggregates can be any shapes, as long as the pacemaker cells within a cell aggregate can transmit electrical stimulation to one another, and may be, for example, sheets, wires, spheres, rods, plates, and other three-dimensional shapes, or amorphous shapes. The shapes of the cell aggregates can be selected as appropriate, depending upon the shape of a desired pacemaker or the shape of an implantation site.

Cell aggregates of the pacemaker cells can be obtained by known methods, such as hanging drop culture, sheet-monolayer culture, and the like.

A cell aggregate(s) which is/are formed by a certain number of the pacemaker cells according to the present disclosure, as an implant, can be implanted to or contacted with the heart by using the aggregate(s) directly, or by supporting the aggregate(s) on sheets or in other matrices, or by placing the aggregate(s) in appropriate containers. The materials of which the sheets and matrices are made include those as exemplified above, and other materials can be also used. In cases where a cell aggregate(s) of the pacemaker cells is/are implanted directly to heart muscle, the implantation may be done either within or on the heart muscle. For example, a cell aggregate(s) of the pacemaker cells may be injected into the heart muscle, or alternatively adhered on the heart muscle. A cell aggregate(s) comprising the pacemaker cells may be used in a similar way as conventional pacemakers by placing the aggregate(s) in an appropriate container.

In a further aspect, the present disclosure provides a cardiac pacemaker comprising, as the essential component, the pacemaker cells disclosed herein, or the implant as described above, the beating rate of which can be controlled by regulation of the Na channel. The cardiac pacemaker may be, for example, one in which the pacemaker cells or a cell aggregate or aggregates comprising the pacemaker cells are supported in a sheet or matrix, or the above-described cell aggregate itself or aggregates themselves, or one in which the pacemaker cells or the above-described cell aggregate or aggregates are placed in an appropriate container. As described above, the beating rate of the pacemaker can be controlled by using Na channel inhibitors or activators, or autonomic stimulants.

It is preferable that a cardiac pacemaker which comprises the pacemaker cells or an implant comprising the pacemaker cells according to the present invention has means for contacting with, or introducing or excreting Na channel inhibitors or activators, or autonomic stimulants. The pacemaker cells or an implant comprising the pacemaker cells according to the present disclosure (for example, a cell aggregate or aggregates) may be contacted directly with the heart or embedded in the heart, such that the above-mentioned drugs can be delivered from the heart. As means for administration of the above-mentioned drugs are exemplified oral administration, intramuscular or intravenous administration by syringe, and the like. These means can be used to introduce Na channel inhibitors or activators, or autonomic stimulants into or removed from the pacemaker cells within the cardiac pacemaker according to the present disclosure, thereby controlling the beating rate.

The present disclosure, in a further aspect, is directed to a pacemaker cell obtained by the present invention or an implant comprising the same, for use in the production of a cardiac pacemaker, the beating rate of which can be controlled by regulation of the Na channel.

Also disclosed is a method for the producing a cardiac pacemaker, the beating rate of which can be controlled by regulation of the Na channel, said method comprising using a pacemaker cell as disclosed herein or an implant comprising the same.

Also described herein is an agent for controlling the beating rate of the pacemaker cell, of the implant or cell sheet, or of the cardiac pacemaker according to the present disclosure, wherein the agent includes Na channel inhibitor or activator. In a further aspect, the present disclosure provides an agent for controlling the beating rate of the pacemaker cell, of the implant or cell sheet, or of the cardiac pacemaker as herein disclosed, wherein the agent includes an autonomic stimulant. Such agents include, but are not limited to, Na channel activators, Na channel inhibitors, sympathetic stimulants, parasympathetic stimulants, and the like, as already exemplified in the specification.

The concentrations of Na channel inhibitor or activator or of autonomic stimulant in the agent, the amounts at which the agent is applied to the pacemaker cell or to the implant or cell sheet, and the amounts at which the agent is applied to the cardiac pacemaker can be readily determined by the physician using routinely available tests. The agent can be not only applied directly to the pacemaker cell or implant according to the present disclosure, but also given to patients by various routes, such as injections, infusions, oral administrations.

Furthermore, the pacemaker according to the present disclosure will not be required to be removed out of the heart, even in cases where the pacemaker has become unnecessary, such as where the heart of a patient has been cured, and in such cases, it is possible that the discontinuation of its function is made by administering a drug which is a Na channel inhibitor, such as lidocaine, mexiletine, and pilsicainide. In cases where pacemaker becomes necessary again, on the other hand, it is possible that its functions are resumed by removing the above-mentioned drug which is a Na channel inhibitor, such as lidocaine, mexiletine, and pilsicainide.

Also disclosed herein is an HCN4 specific antibody, which is generated against an amino acid sequence of an extracellular domain of HCN4, preferably VSINGMVNNSW (SEQ ID NO:1) or VPMLQDFPHD (SEQ ID NO:2). In embodiments, cysteine can be added at either terminus of these sequences to serve binding to carrier proteins. For example, such cysteine-added sequences include amino acid sequences VSINGMVNNSWC (SEQ ID NO:3) and CVPMLQDFPHD (SEQ ID NO:4). An antibody thus obtained is one which recognizes as the antigen recognition site an amino acid sequence of an extracellular domain of HCN4, preferably an amino acid sequence set forth in SEQ ID NO:1 or SEQ ID NO:2. Those skilled in the art can obtain antibodies generated not only against the above-mentioned amino acid sequences, but also against amino acid sequences of other portions of HCN4, using procedures as described above.

HCN4 specific antibodies can be used in the method for obtaining a pacemaker cell described above. The HCN4 specific antibodies which can be used in the method for obtaining a pacemaker cell, but are limited to, antibodies as described above. The HCN4 specific antibody can be used with a detectable label attached thereto, thereby to obtain efficiently a pacemaker cell according to the present disclosure.

The present invention will be described in a detailed and specific manner below by way of Examples, which should in no way be construed to limit the present invention.

### [Examples]

### Example 1

### Production of pacemaker cells

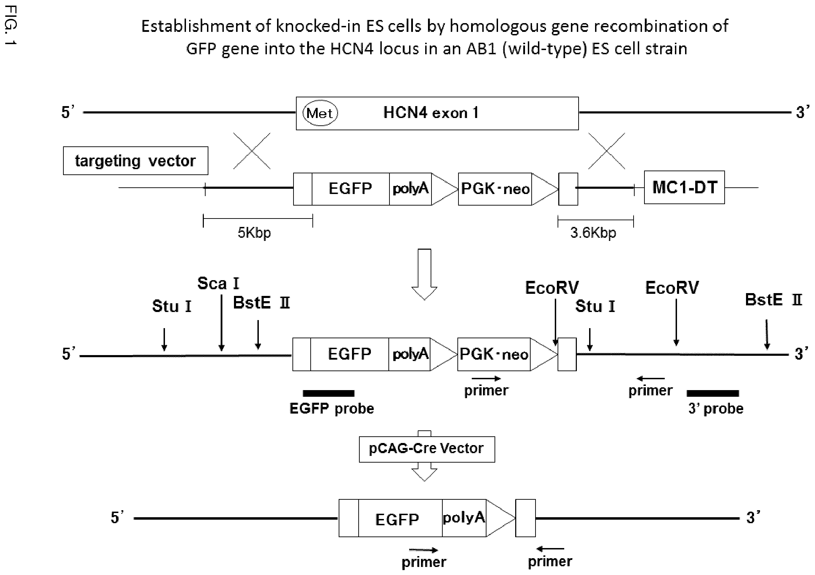

As shown in Fig. 1, a targeting gene was constructed by connecting a selection/collection label, a GFP protein gene, into the promoter region of HCN4 channel gene. Specifically, a targeting vector was designed so that an EGFP+PGK-neo unit was inserted at the position of the first Met codon of exon 1 of the HCN4 gene, thereby to delete a portion of the coding region. The resulting targeting vector was subjected to gene introduction into 90%-confluent AB1 ES cells by electroporation.

Twenty-four hours after the introduction of the targeting gene, the medium was replaced, and Geneticin was added to the medium to 250 µg/ml, followed by selection for about one week. Colonies resulting from the selection were collected on feeder cells in 96-well plates. Which of the obtained clones had the gene insertion was verified with PCR. 10 µg of genome DNA from each of the clones collected after the selection was digested overnight with a restriction endonuclease (BstE II), and then subjected to electrophoresis on 1.0% agarose gel having EtBr added at 0.5 µg/ml, for about 5 hours under conditions of 100V and 80A. The DNA was denatured in alkaline transfer buffer (0.4 M NaOH, 1M NaCl), and then transferred onto nylon membrane (Amersham Biosceinces). Hybridization was performed overnight with a probe which had been labeled with α-³²P (Institute Isotopes) using Bca BEST Labeling Kit (TaKaRa). The membrane after hybridization was washed sequentially in 2X SSC, 0.5% SDS; 2X SSC, 0.1% SDS; 0.2X SSC, 0.1% SDS, twice; and 0.2X SSC. The radioactivity was measured to detect the DNA sequence of interest.

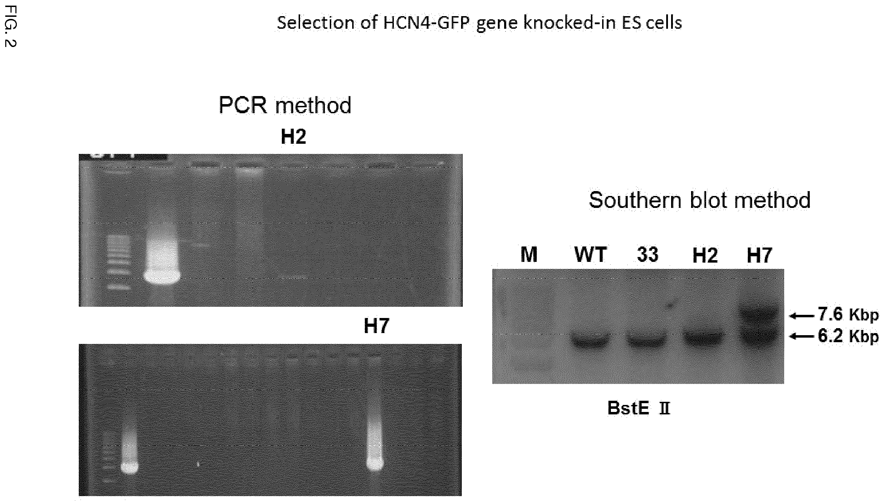

As shown in Fig. 2, knocked-in ES cells could be established in which the GFP gene had been incorporated into an allele on one chromosome by introducing the targeting gene into ES cells. The establishment of these knocked-in ES cells was verified by PCR and southern blot methods.

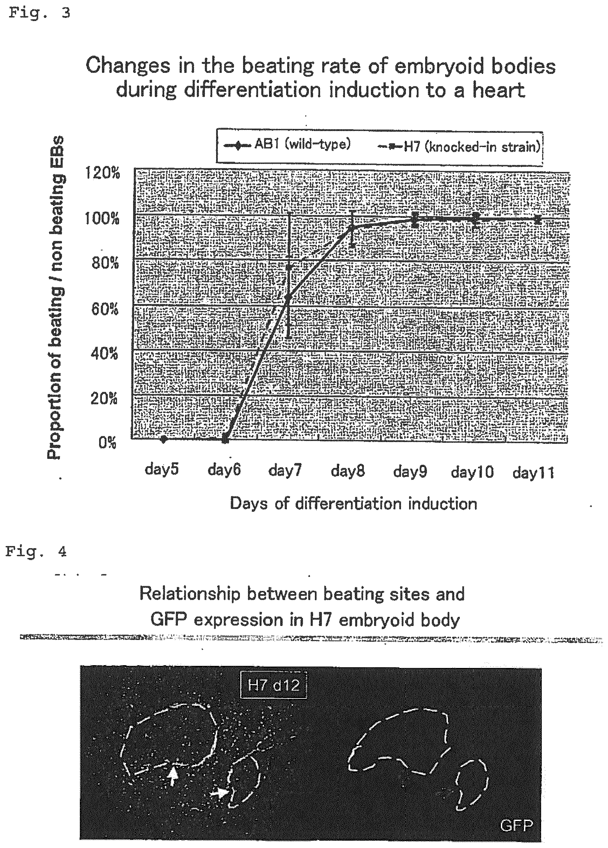

Subsequently, feeder cell-free knocked-in ES cells, which were obtained by plating the above-described knocked-in ES cells in gelatin-coated dishes for about one hour to remove feeder cells from the undifferentiated ES cells, were suspended to about 25,000 cells/ml in differentiation medium (DMEM, FBS, PSG, MEM non-essential amino acids solution, sodium pyruvate, 2-ME). Droplets of 20 µl were placed in several rows on a dish with an 8-channel pipetter, and the dish was inverted for carrying out three-dimensional culture (hanging drop culture) to form EBs, which were cell aggregates. The day on which EBs begun to form was defined as day 0 of differentiation induction. The differentiation medium was added on day 2 of induction, followed by floating culture. Day 6, EBs were allowed to adhere on gelatin-coated dishes. On the next day, the medium was replaced. As shown in Fig. 3, once knocked-in ES cells formed embryoid bodies, beating embryoid bodies were increased in number over time, and exhibited cardiac differentiation equivalent to that in a wild-type strain.

GFP signals at beating sites in embryoid bodies (day 12) were observed with an incubation imaging system, which can make observation of living embryoid bodies. As shown in Fig. 4, portions of the embryoid body were beating, in accordance with the sites where the signal from GFP was detected.

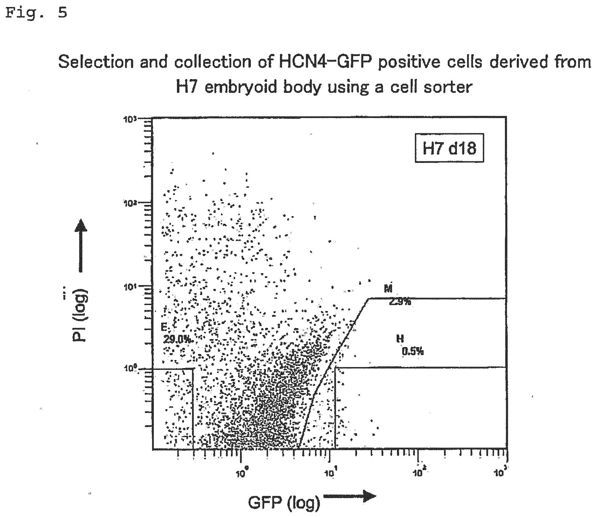

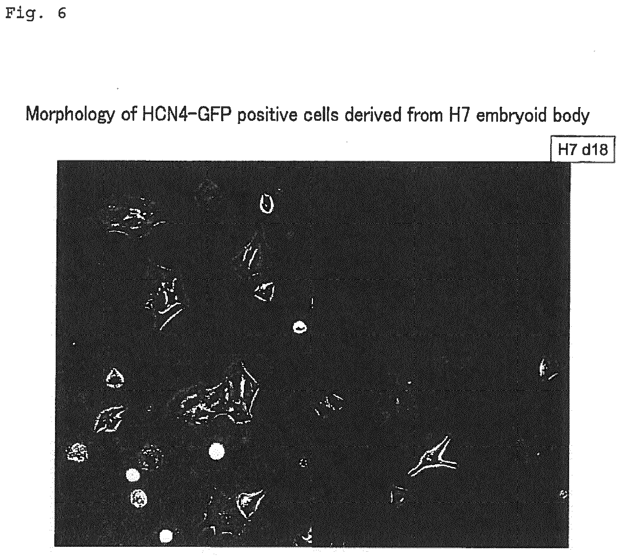

Only cells positive for GFP signal were selected and collected by means of FACS. Embryoid bodies formed were subjected to trypsin treatment (in 1X trypsin/EDTA/GIBCO-BRL at 37°C for 5 to 15 minutes) to dissociate them into single cells. For embryoid bodies on or after day 10 of differentiation induction, in addition to the trypsin treatment, collagenase treatment was carried out (collagenase type II, 37°C, one hour). Cells obtained were suspended in HBSS-1% FCS (HBSS: Hank's Balanced Salt Solution, CAMBREX), 2.5 µg/m propidium iodide (PI). GFP(+)/PI(-) cells within the embryoid bodies were collected using a cell sorter (BECKMAN-COULTER EPICS ALTRA) using AB1 or COS7 cells as a negative control. As shown in Fig. 5, GFP positive cells accounted for 0.5% of the total number of cells. Fig. 6 shows GFP-signal positive cells which were selected and collected using FACS.

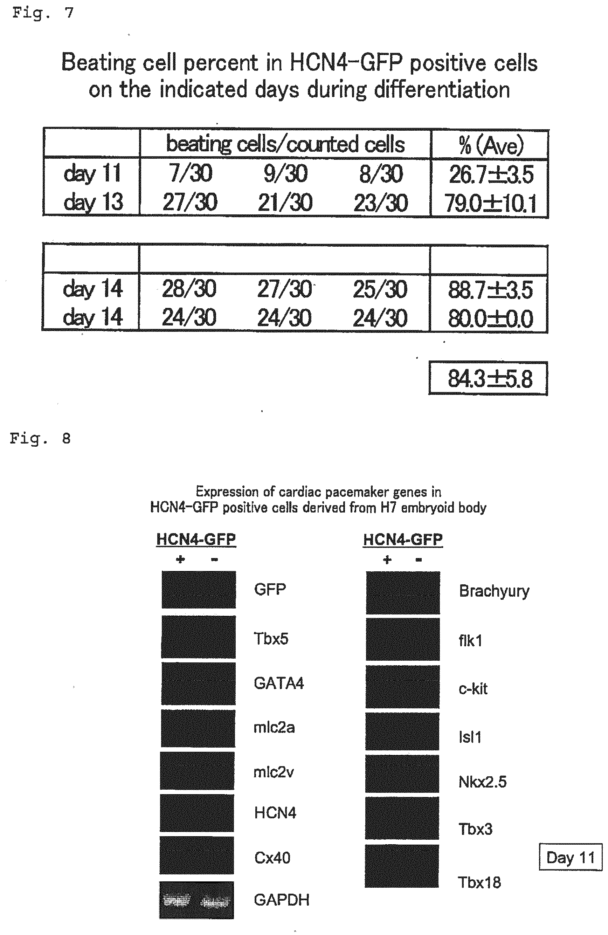

Cells, which had been collected by cell sorting, were seeded on gelatin-coated dishes in a number of about 5,000 cells. On the next day, observations were made for percentages of beating cells in the collected cells, and their morphology, size, and the like under an optical microscope to examine the relationship with beating. As shown in Fig. 7, the GFP-signal positive cells had a beating percentage of 85%.

Gene expression in GFP positive cells was examined by the following experimental procedures.

RNA extraction: Using an HT7 strain, undifferentiated ES cells and embryoid bodies on days 0 to 21 were collected at fixed time points, and then dissolved in buffer RLT, followed by RNA extraction (QIAGEN RNeasy Micro Kit, QIAGEN). DNase treatment (TaKaRa Dnase I (RNase-free)) was performed at 37°C for 30 minutes, to prevent genomic DNA contamination. For Nkx2.5-GFP positive cells using an HCGP7 strain, after sorting on a cell sorter, cells were washed in 1X PBS, and then treated in a similar way.

RT-PCR: The RNA obtained by the above-described procedure was used to prepare reaction solutions containing: 1 µl of Oligo dT primer (50 µM), 1 µl of dNTP mixture (10 mM each), 0.5 µg of template RNA, and RNase-free dH₂O (distilled water) up to 10 µl. The reaction solutions were incubated for 5 minutes at 65°C, and then cooled on ice. To each of these template RNA/primer mixtures were added 4 µl of 5X Primescript® buffer, 0.5 µl of RNase inhibitor (40 U/µl), 1 µl of Primescript® RTase (200 U/µl), and 4.5 µl of RNase-free dH₂O, and then incubated for 60 minutes at 42°C to synthesize cDNA (PrimeScript® 1st strand cDNA synthesis Kit, TaKaRa). The cDNA synthesized was used to perform PCR (TaKaRa Ex Taq® Hot Start Version). The conditions for PCR were: 25 to 30 cycles of denaturing at 94°C for 30 seconds, annealing at 60°C for 1 minute, and extension at 72°C for 30 seconds. The sequence of the gene-specific primers used in RT-PCR is given in Table 1.

**[Table 1]**

| Table 1: Primers used in RT-PCR | | |
|---|---|---|
| Name of gene | | Sequence (5'→3') |
| GAPDH | forward | TGAACGCGAAGCTCACACTGG: SEQ ID NO:5 |
| | reverse | TCCACCACCCTGTTGCTGTA: SEQ ID NO:6 |
| Oct3/4 | forward | AGATCACTCACATCGCCAAT: SEQ ID NO:7 |
| | reverse | AAGGTGTCCTGTAGCCTCAT: SEQ ID NO:8 |
| Nanog | forward | GCAAGAACTCTCCTCCAT: SEQ ID NO:9 |
| | reverse | ATACTCCACTGGTGCTGA: SEQ ID NO::10 |
| Brachyury | forward | CATTACACACCACTGACGCA: SEQ ID NO:11 |
| | reverse | CATAGATGGGGGTGACACAG: SEQ ID NO:12 |
| GATA-4 | forward | CTGCGGCCTCTACATGAAGC: SEQ ID NO:13 |
| | reverse | TCTTCACTGCTGCTGCTGCT: SEQ ID NO:14 |
| Nkx 2.5 | forward | CAAGTGCTCTCCTGCTTTCC: SEQ ID NO:15 |
| | reverse | GGCTTTGTCCAGCTCCACT: SEQ ID NO:16 |
| MEF2c | forward | CCCTTCGAGATACCCACAAC: SEQ ID NO:17 |
| | reverse | TGCCCATCCTTCAGAGAGTC: SEQ ID NO:18 |
| Flk-I | forward | ATGACAGCCAGACAGACAGT: SEQ ID NO:19 |
| | reverse | GGTGTCTGTGTCATCTGAGT: SEQ ID NO:20 |
| Isl-I | forward | CTGCAGCCGACAGCTCAT: SEQ ID NO:21 |
| | reverse | CTGCCTAGCCGAGATGGGTT: SEQ ID NO:22 |
| Tbx3 | forward | GAGATGGTCATCACGAAGTC: SEQ ID NO:23 |
| | reverse | GGAAGGCCAAAGTAAATCCG: SEQ ID NO:24 |
| c-kit | forward | CAGAATCGTGGGACCCAT: SEQ ID NO:25 |
| | reverse | CGGCGTCCAGGTTTCTAG: SEQ ID NO:26 |
| Mesp-I | forward | CAGAATCGTGGGACCCAT: SEQ ID NO:27 |
| | reverse | CGGCGTCCAGGTTTCTAG: SEQ ID NO:28 |
| Mlc2-v | forward | AAGGTGTTTGATCCCGAGGG: SEQ ID NO:29 |
| | reverse | GGGAAAGGCTGCGAACATCT: SEQ ID NO:30 |
| Mlc2-a | forward | TGACCCAGGCAGACAAGTTC: SEQ ID NO:31 |
| | reverse | CGTGGGTGATGATGTAGCAG: SEQ ID NO:32 |
| HCN1 | forward | CTCCACTTTGATCTCCAGAC: SEQ ID NO:33 |
| | reverse | TTCTGCATCTGGGTCTGTAT: SEQ ID NO:34 |
| HCN2 | forward | GACAATTTCAACGAGGTGCT: SEQ ID NO:35 |
| | reverse | CCATCTCACGGTCATATTTG: SEQ ID NO:36 |
| HCN3 | forward | AACCCTCCATGCCAGCCTAT: SEQ ID NO:37 |
| | reverse | CTTCCAGAGCCTTTACGCCT: SEQ ID NO:38 |
| HCN4 | forward | CGACAGCGCATCCATGACTA: SEQ ID NO:39 |
| | reverse | GCTGGAAGACCTCGAAACGC: SEQ ID NO:40 |
| Cav3.2 | forward | GCTGTTTGGGAGGCTAGAAT: SEQ ID NO:41 |
| | reverse | CGAAGGTGACGAAGTAGACG: SEQ ID NO:42 |
| Connexin 30.2 | forward | CTCAGCTCTAAGGCCCAGGTCCCG: SEQ ID NO:43 |
| | reverse | CCGCGCTGCGATGGCAAAGAG: SEQ ID NO:44 |
| Connexin 40 | forward | CTCCTCCCCCTGACTTCAAT: SEQ ID NO:45 |
| | reverse | CGCCGTTTGTCACTATGGTA: SEQ ID NO:46 |

For single cells, the following procedures were used.

Reaction solution were prepared by combining 1 µl of Primescript 1step enzyme mix, 1 µl of 2X 1-step buffer, 2 µl of gene-specific primer (20 µM), 1 µl of template cDNA, and RNase-free dH₂O up to 50 µl. These reaction solutions were subjected to cDNA synthesis and PCR under the following conditions (PrimeScript One Step RT-PCR Kit Ver. 2, TaKaRa).

Reverse transcription reaction was carried out at 50°C for 30 minutes, followed by denaturing of reverse transcriptase at 94°C for 2 minutes and then 35 to 43 cycles of denaturing at 94°C for 30 seconds, annealing at 60°C for 1 minute, and extension at 72°C for 30 seconds. PCR products were subjected to electrophoresis on 2.0% agarose gel (Nusieve 3:1 agarose, CMR). For primers from which no PCR products were verified on the agarose gel, PCR reactions were subjected to electrophoresis on 10% acrylamide gel (150 V, 180 mA, 70 minutes), followed by silver staining to detect bonds (PlusOne DNA Silver Staining Kit, GE Healthcare).

As shown in Fig. 8, GFP-signal positive cells expressed HCN4 channel gene and additionally cardiac muscle specific genes such as Tbx5, GATA4, mlc2a, mlc2v, and the like.

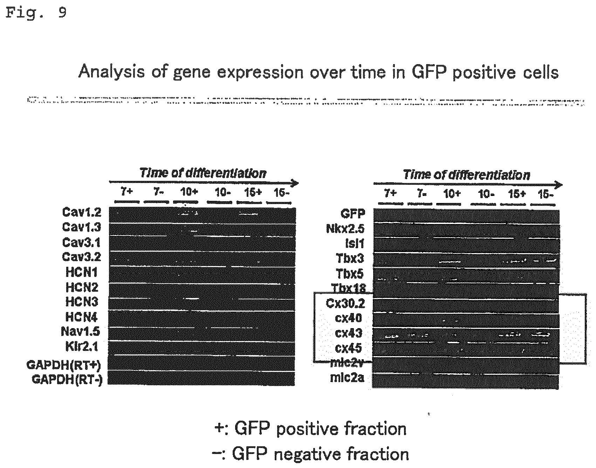

Further, examination was made of the expression of various genes in GFP-signal positive cells over time (on days 7, 10, and 15 of differentiation). The results are shown in Fig. 9. It was found that the expression of the HCN gene and other cardiac muscle specific genes tended to increase over time. It was also found that in a fraction of GFP positive cells, the expression of Cx30.2, Cx40, Cx43, and Cx45 tended to increase over time (the genes boxed in Fig. 9), and it turned out that the functions of transmitting electrical stimulation of the cells were enhanced with differentiation.

Channels expressed in GFP positive cells were also examined by the following experimental procedures. Immunostaining

Cells were fixed in 4% paraformaldehyde at room temperature for 30 minutes, washed three times in PBS, and placed in 0.2% Triton X-100/PBS for 10 minutes, followed by washing twice in PBS. After that, the cells were blocked in 5% skim milk/PBS at room temperature for 30 minutes. Then, the cells were further washed three times in PBS and subjected to reaction with a primary antibody diluted in 0.1% tween-20/1% BSA/HBSS (BSA: bovine serum albumin, SIGMA) at room temperature for 30 minutes and then to reaction with a secondary antibody in the dark at room temperature for 30 minutes, followed by washing three times in PBS, and then RNase treatment, and finally nuclear staining with DAPI (Wako).

The primary antibodies used were an anti-HCN4 antibody produced, an anti-HCN4 rabbit polyclonal antibody (Osenses), a monoclonal anti-tropomyosin (Sarcomeric) clone CH1 (SIGMA), an anti-Nav1.5 rabbit polyclonal IgG (abcam), an anti-Kir2.1 rabbit polyclonal IgG (alomone labs), and a purified rat anti-mouse CD31 (BD Pharmingen). The secondary antibodies used were an Alexa Flulor 568 goat anti rabbit IgG(H+L), an Alexa Flulor 568 goat anti rat IgG(H+L), and an Alexa Flulor 546 goat anti mouse IgG(H+L) (Molecular Probes).

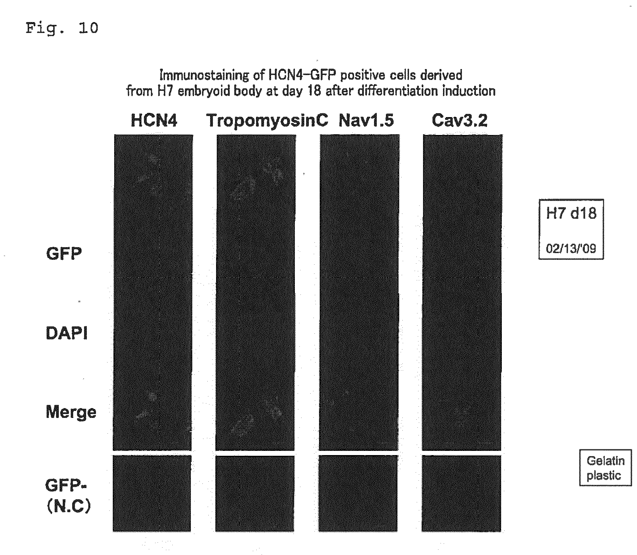

As shown in Fig. 10 (and in Fig. 9), GFP-signal positive cells expressed not only HCN4 channel, but also Cav3.2 channel, a channel specific for pacemaker cells. In addition, Na channel (Nav1.5) was strongly expressed.

On GFP-signal positive cells, action potential and HCN channel activities were examined by the following experimental procedures.

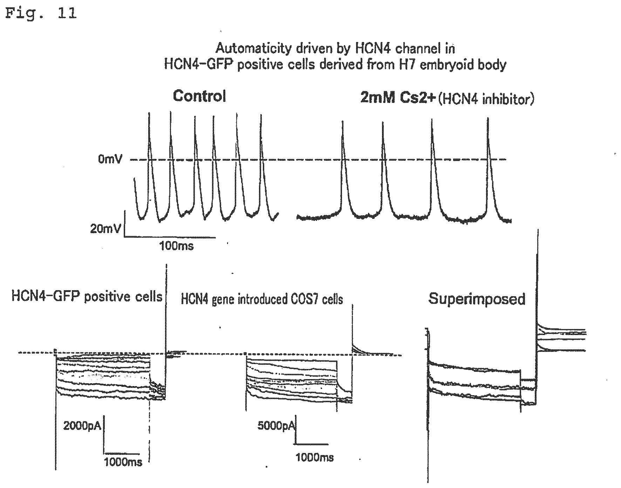

Patch clamp: Cells which had been collected by cell sorting were seeded on gelatin-coated, 8-mm diameter cover slips (Warner Instruments) in a number of about 1,500 cells. On or after the next day, a patch clump method was used to observe the automaticity and to analyze functions of ion channels. As shown in Fig. 11, GFP-signal positive cells generated spontaneous action potential and had HCN channel activity.

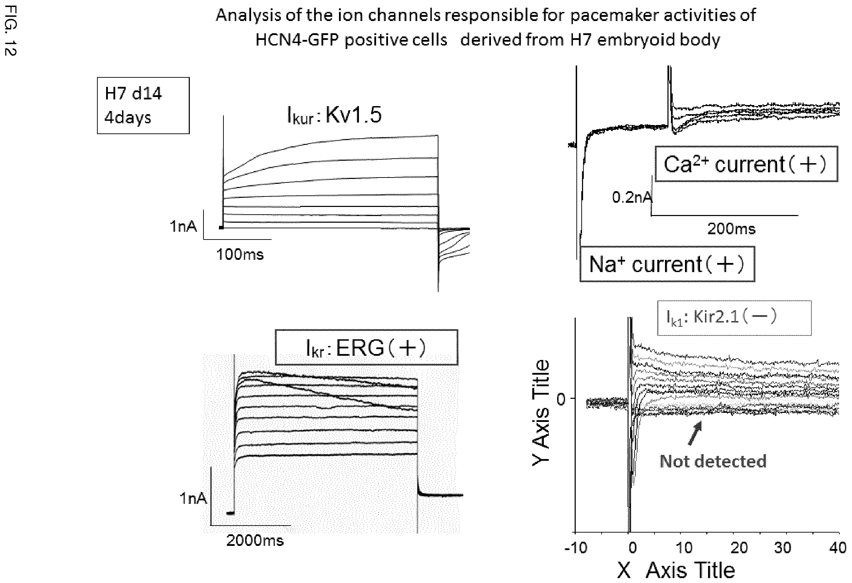

Furthermore, on GFP-signal positive cells, a patch clamp method similar to the above was used to observe the automaticity and to analyze functions of ion channels. As shown in Fig. 12, GFP-signal positive cells not only expressed the ERG and Ca channels necessary for pacemaker activities, and strongly had Na channel activity.

### Example 2

### Control of the beating rate of the pacemaker cells

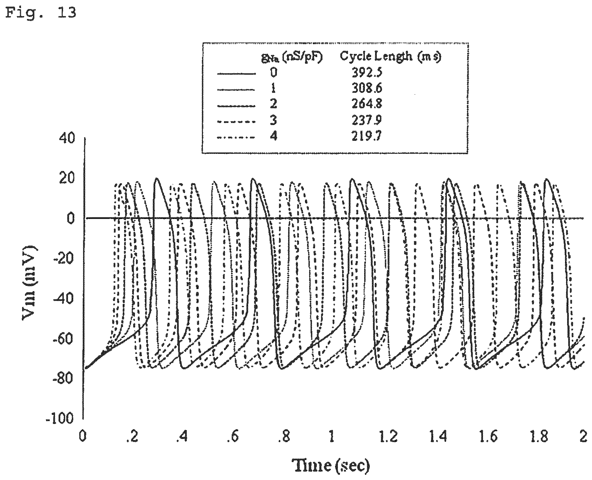

Electrical activities were simulated when Na channel was blocked, using a pacemaker cell model described in Kurata Y, Matsuda H, Hisatome I, Shibamoto T., Effects of pacemaker currents on creation and modulation of human ventricular pacemaker: theoretical study with application to biological pacemaker engineering. Am J Physiol Heart Circ Physiol. 2007 Jan; 292(1) :H701-18. As shown in Fig. 13, it turned out that when Na channel was controlled in the simulations, the GFP-signal positive cells were able to change the beating rate.

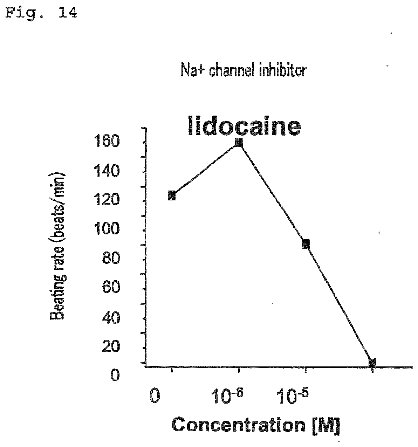

Subsequently, experiments were carried out which demonstrated that controlling Na channel could result in actually controlling the beating rate of the GFP-signal positive cells described above. The quantification of electrical activities by a patch clump method and of contraction by video recording was carried out. As shown in Fig. 14, it turned out that the GFP-signal positive cells were actually able to control the beating rate by means of Na channel inhibitors.

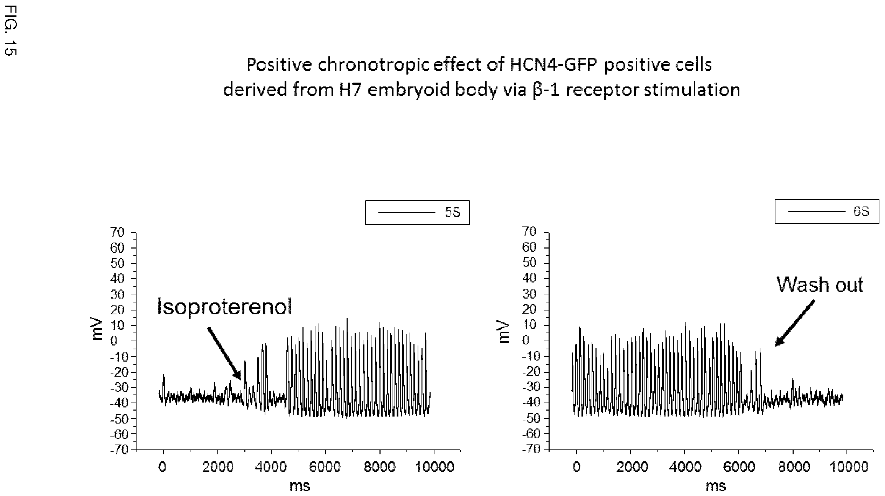

Additional experiments were conducted which demonstrated that the GFP-signal positive cells was able to increase the beating rate by means of autonomic stimulants. The quantification of electrical activities by a patch clump method was carried out. As shown in Fig. 15, it turned out that the GFP-signal positive cells were able to increase the beating rate by means of autonomic stimulants.

It was ascertained that the beating rate of the pacemaker cells was increased by means of isoproterenol, a sympathetic stimulant, and decreased by means of carbachol, a parasympathetic stimulant. In these experiments, a patch clamp method was used to measure directly electrical phenomena (spontaneous excitation) from the pacemaker cells. To the perfusion solution were added isoproterenol, a sympathetic stimulant, which increased [sic], and then carbachol, a parasympathetic stimulant, to examine their effects. It was observed that washing out of these respective drugs resulted in the disappearance of their effects.

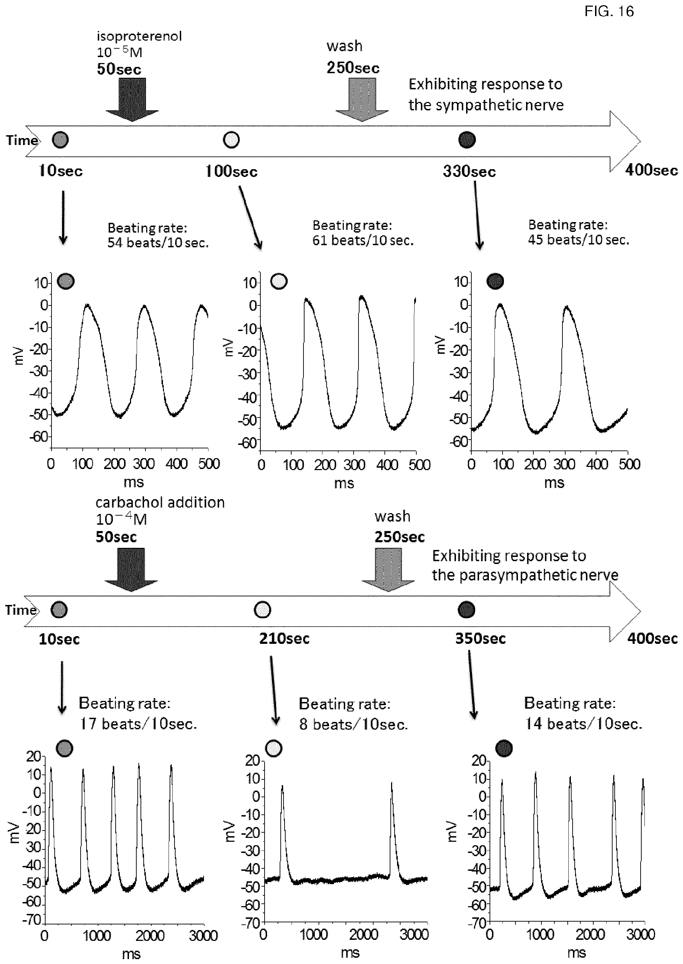

As shown in Fig. 16, the beating rate was changed before and after the presence of 10⁻⁵ M isoproterenol. Isoproterenol increased the beating rate from 54 beats per 10 seconds to 61 beats per 10 seconds, and washing out of isoproterenol resulted in a decrease in the beating rate to 45 beats per 10 seconds. Subsequently, the presence of carbachol decreased the beating rate to 17 beats per 10 seconds to 8 beats per 10 seconds, and when carbachol was washed out, the beating rate exhibited an increasing tendency with a beating rate of 14 beats per 10 seconds.

### Example 3

### Implantation of the pacemaker cells to the heart with atrioventricular block

### (1) Culturing of pacemaker cells

Cells used were AB1 cells, which are 129SV/EV mouse derived ES cells (kindly given by Dr. Shimono, RIKEN [The Institute of Physical and Chemical Research]), a strain HCN4-EGFP-AB1 (#33), which was established by gene knock-in of a plasmid pXNL-HCN4-EGFP in AB1 cells, and cells sorted by cell sorting using the fluorescence from GFP as a marker (HCN4-cos7). Cells of each of the strains AB1 and #34 were cultured on mitomycin C-treated SNL cells (which are STO-derived, forced to express LIF, and neomycin resistant).

Passage of ES cells was continued by culturing them as follows: after washing in 1X PBS, cells were detached form dishes with 0.25% trypsin/EDTA (GIBCO-BRL), suspended for dilution, and seeded on new dishes (or new SNL cells treated with mitomycin C (SIGMA) for the strains AB1 and #34). The composition of maintenance medium was as follows:
Dulbecco's Modified Eagle's Medium(DMEM Sigma) for AB1 and #33, bovine serum (FBS JRH), penicillin-streptomycin-L-glutamine sodium (SIGMA), MEM non-essential amino acids solution (GIBCO-BRL), sodium pyruvate (SIGMA), 0.1 mM 2-mercaptoethanol (SIGMA), LIF (ESGRO/Cosmo Bio).

Culture used 0.1% gelatin (SIGMA)-coated, 100-mm diameter dishes (FALCON) and carried out at 37°C under 5% CO₂.

### (2) Formation of embryoid bodies

Formation of embryoid bodies was performed by preparing hanging drops of 20 µl differentiation medium adjusted such that about 500 cells were present, which were then subjected to inverting and culturing. The composition of differentiation medium was as follows: DMEM, FBS, penicillin-streptomycin-L-glutamine sodium (100X), MEM non-essential amino acids solution, sodium pyruvate, 2ME.

The day of preparing the hanging drops was set to be day 0, and differentiation medium was added on day 2. In cases where cell sorting was carried out in an early period of differentiation induction, floating culture was continued. In cases where cell sorting was carried out in a middle or late period of differentiation induction, cultures were transferred to gelatin-coated dishes on day 4 and culturing under adhesive conditions was continued. Embryoid bodies were used for immunostaining after sorting GFP positive cells by cell sorting.

### (3) Cell sorting

Embryoid bodies formed were trypsin treated (in 1X trypsin/EDTA (GIBCO-BLR) at 37°C for 5 to 15 minutes) to dissociate cells into single cells. In cases of embryoid bodies on or after day 10 of differentiation induction, in addition to the trypsin treatment, collagenase treatment was carried out (collagenase type II, 37°C, one hour). Cells obtained were suspended in HBSS-1% FCS (HBSS: Hank's Balanced Salt Solution, CAMBREX), 2.5 µg/ml propidium iodide (PI). GFP(+)/PI(-) cells within the embryoid bodies were collected using a cell sorter (BECKMAN-COULTER EPICS ALTRA) using AB1 or COS7 cells as a negative control.

### (4) Generation of model rats with AVB (AtrioVentricular Block) as a bradyarrhythmia model

Male ICR rats of 10 weeks old were used. Rats were administered with a 0.2 ml injection of an anesthetic (0.2% pentobarbital, MiliQ). Breathing assistance was provided by a mechanical ventilator and electrocardiogram monitoring was performed. A thoracotomy was made using an electrosurgical knife and 15 µl of 30% glycerol was injected using a microsyringe, into a place where the pulmonary artery is attached to the heart. It was ascertained, from the electrocardiogram, whether AVB wave forms (decrease in the beating rate, independent P and QRS waves) appeared when the injection allowed the atrioventricular node to be damaged. If AVB wave forms were not identified, injection of glycerol was done again. Thirty minutes after AVB wave forms were identified, the chest was closed after AVB wave forms were identified on the electrocardiogram. The ventilator was removed from each of the rats, which were subjected to oxygen inhalation at 37°C. When recovered from anesthesia, the rats were returned back to their cages.

Five days post-surgery, electrocardiograms of the rats were examined to ascertain whether occurrence of P wave was independent from that of QRS waves.

### (5) Introduction of the MiwZ gene for immunostaining of biological pacemaker cells

The MiwZ gene was knocked-into A6 cells by electroporation. Grown colonies were subjected to X-gal staining in their undifferentiated states, thereby selecting four strains which displayed intense staining. These strains were subjected to differentiation induction. Pacemaker cells which were GFP positive cells were sorted by cell sorting and verified to be stained with X-gal.

### (6) Organization and implantation of biological pacemaker cells

Pacemaker cells which were GFP positive cells were selected and collected by cell sorting. The pacemaker cells were collected in conical tubes, and then centrifuged at 1,000 rpm for 5 minutes to form a cell pellet, which was then suspended in differentiation induction medium.
Hanging drops adjusted such that 3,000 cells were present in 20 microliters were prepared and three-dimensional culture in the hanging drops was performed. After 48 hours, 30 cell-aggregates were combined together and implanted at the right ventricular apex of a bradycardic model rat through a needle.

Five days after surgery, a rat which had an electrocardiogram identifying atrioventricular block wave forms was administered with a 0.2 ml injection of an anesthetic (0.2% pentobarbital, MiliQ). Breathing assistance was provided by a mechanical ventilator and electrocardiogram monitoring was performed. A thoracotomy was made using an electrosurgical knife and an assembly of combined aggregates suspended in 20 µl PBS was injected into the ventricular wall using a microsyringe. The chest was closed and the ventilator was removed from the rat, which was subjected to oxygen inhalation at 37°C. When recovered from anesthesia, the rat was returned back to its cage. Five day later, the chest was opened, and the heart was removed and fixed in 4% paraformaldehyde-PBS.

### (7) Advantages of organizing pacemaker cells

When pacemaker cells which were GFP positive cells were selected and collected by cell sorting, and cultured as single cells in the differentiation induction medium, the cells depolarized in one week and lost function as pacemaker cells. On the other hand, it was possible that in cases where aggregates were formed by preparing hanging drops adjusted such that 3,000 cells were present in 20 microliters and performing three-dimensional culture in the hanging drops, the cells retained function as pacemaker cells even after one month in culture dishes.

In these experiments, the pacemaker cells could exhibit pacemaker capabilities after implantation by implanting 30 aggregates, each of which comprised about 3,000 cells, to the ventricular wall of a bradycardic model rat. Further, the pacemaker cells could exhibit pacemaker activities after implantation, also in cases of gathering 5 cell-aggregates, each of which comprised about 3,000 cells of the pacemaker cells, to form a larger cell-aggregate and implanting 6 of these larger tissue-aggregates to the ventricular wall of a bradycardic model rat. In addition, the pacemaker cells could exhibit pacemaker activities after implantation, also in cases of gathering 5 cell-aggregates, each of which comprised about 3,000 cells of the pacemaker cells, to form a larger cell-aggregate and implanting 4 of these larger tissue-aggregates to the ventricular wall of a bradycardic model rat. When 15 aggregates, each of which comprised about 3,000 cells, were implanted, however, the pacemaker cells were not able to exhibit pacemaker activities.

### (8) Evaluation of intraventricular engraftment of pacemaker cells by X-gal staining

The heart was fixed in 1% glutaraldehyde for 30 minutes, and then washed three times,10 minutes each, in 10 ml of detergent rinse (0.1 M phosphate buffer, pH 7.3, 3.2 mM MgCl₂, 0.01% sodium deoxycholate, 0.02% Nonidet P-40, 0.02M Tris buffer, pH 7.3). Staining was carried out overnight at 37°C in the dark using 4 ml of staining solution (0.1 M phosphate buffer, pH 7.3, 3.2 mM MgCl₂, 0.01% sodium deoxycholate, 0.02% Nonidet P-40, 5 mM K₄[Fe(CN)₆], 5 mM K₃[Fe(CN)₆]).

### (9) Changes in electrocardiogram of rats with implanted pacemaker cells

First, a thoracotomy was made under anesthesia to prepare rats with atrioventricular block using glycerol. Then, cell aggregates containing organized pacemaker cells were implanted by their injection into the myocardial wall. The pacemaker cells had the X-gal gene introduced therein, and thus could be stained by X-gal staining. In the pictures, blue-stained cells are implanted cells, from which it is verified that the tissue segments were engrafted (Fig. 17). On 7 days after the atrioventricular block was caused, it was ascertained that the rats with atrioventricular block survived and the atrioventricular block remained existing (after atrioventricular block in Fig. 18), and saline was injected as a control to compare with the implants containing the pacemaker cells. In electrocardiograms, a ventricular rhythm with wider wave forms was brought about in the case where the implants containing the pacemaker cells were implanted, whereas narrower ventricular wave forms were visible in the case of the saline control.

Fig. 19 verifies, in the second row electrocardiogram from the top, that the atrioventricular block remained existing and shows that the ventricular rhythm with wider wave forms was brought about in the case where the implants containing the pacemaker cells were implanted (see, the third row electrocardiogram from the top). Based on these results, isoproterenol, which is a sympathemic stimulant, was injected into the peritoneal cavity. It was ascertained that a ventricular rhythm with an increased pulse rate and wider wave forms, a rhythm resulting from the pacemaker cells, was accelerated (tachycardia).

### Example 4

### Production of HCN4 specific antibodies

Production of antibodies was outsourced to MBL (Molecular & Biological Laboratories). As an antigen was selected the HCN4 protein which constitutes the If channel specific for pacemaker cells. In order to allow labeling from the outside of the pacemaker cells, two amino acid sequences, corresponding to a portion of the pore region which is exposed outside the cell (VSINGMVNNSW, SEQ ID NO:1) and a portion from within the pore region and into an extracellular region (VPMLQDFPHD, SEQ ID NO:2) were used to synthesize peptides in which cysteine was added for binding to carrier proteins (VSINGMVNNSWC (SEQ ID NO:3) and CVPMLQDFPHD (SEQ ID NO:4)). Using these peptide, three rabbits per peptide were immunized (rabbits #1 to #3 were immunized with SEQ ID NO:3, and rabbits #4 to #6 with SEQ ID NO:4). After eight immunizations, each of the rabbits was subjected to taking whole blood to collect serum, thereby to obtain an antibody.

Antibody purification was carried out as follows: the proteins in the serum were fractionated by ammonium sulfate precipitation, followed by removing the ammonium sulfate by passing through a column (PD-10 Desalting Column, GE Healthcare). A protein-G column (HiTrap Protein G HP, GE Healthcare) was filled with 20 mM sodium phosphate (pH 7.0) and the protein solution was loaded onto the column. The column was washed with a solution of sodium phosphate, and then eluted with 0.1 M glycine HCl (pH 3.0). The collected eluate was neutralized with 1M Tris-HCl (pH 8.8), and then concentrated by centrifugation in filter-attached tubes (Amicon Ultra, Millipore). From ELISA results, antibodies from rabbits #3 and #5 were subjected to affinity purification on an antigen column.

As shown in Fig. 20, novel antibodies specific for an extracellular domain of HCN4 were able to be generated.

The obtained antibodies were verified to recognize HCN4 by the following experiments.

Western blotting: cos 7 and HCN4-EGFP-cos7 cells were lysed in SDS-Sample Buffer (Laemmli Sample Buffer, BIO RAD), 2-mercaptoethanol (WAKO), and the resulting samples were applied to 7.5% acrylamide gel at a volume of 10 ul per lane and electrophoresed at 80 V in the stacking gel and after entering the separating gel, at 100 V for another 90 minutes. The gel after electrophoresis was subjected to protein transfer onto a membrane by a semi-dry method under conditions of 0.18 A for 30 minutes. After the protein transfer, the membrane was blocked in 5% skim milk-TBST (for 3 hours at room temperature or overnight at 4°C), and then subjected to reaction with a diluted primary antibody (an anti-HCN4 antibody produced or commercial available anti-GFP antibody) at room temperature for 30 minutes and then to reaction with a diluted secondary antibody (an HRP-conjugated anti-rabbit IgG antibody or anti-mouse IgG antibody) at room temperature for 30 minutes, followed by washing three times in TBST and ECL detection, comparing detection capability of each antibody. As shown in Fig. 21, it turned out that the antibody strongly recognized HCN4.

Next, it was ascertained that the antibody was able to recognize HCN4 on the cell membrane of the GFP-signal positive cells from the outside of the cells. Experimental methods and procedures were as follows.

Immunostaining: cells were fixed in 4% paraformaldehyde/PBS at room temperature for 30 minutes, washed three times in PBS, and placed in 0.2% Triton X-100/PBS for 10 minutes, followed by washing twice in PBS. After that, the cells were blocked in 5% skim milk/PBS at room temperature for 30 minutes. Then, the cells were further washed three times in PBS and subjected to reaction with a primary antibody diluted in 0.1% tween-20/1% BSA/HBSS (BSA: bovine serum albumin, SIGMA) at room temperature for 30 minutes and then to reaction with a secondary antibody in the dark at room temperature for 30 minutes, followed by washing three times in PBS, and then RNase treatment, and finally nuclear staining with DAPI (Wako).

The primary antibodies used the anti-HCN4 antibody produce, an anti-HCN4 rabbit polyclonal antibody (Osenses), a monoclonal anti-tropomyosin (Sarcomeric) clone CH1 (SIGMA), an anti-Nav1.5 rabbit polyclonal IgG (abcam), an anti-Kir2.1 rabbit polyclonal IgG (alomone labs), and a purified rat anti-mouse CD31 (BD Pharmingen). The secondary antibodies used an Alexa Flulor 568 goat anti rabbit IgG(H+L), an Alexa Flulor 568 goat anti rat IgG(H+L), and an Alexa Flulor 546 goat anti mouse IgG(H+L) (Molecular Probes).

As shown in Fig. 22, it turned out that the antibody was able to recognize HCN4 on the cell membrane of the GFP-signal positive cells from the outside of the cells.

### [Industrial Applicability]

The present invention can be used in the field of medical devices, especially in the field of cardiac pacemakers.

### [Sequence Listing Free Text]

SEQ ID NO:1 represents the amino acid sequence of a portion of an extracellular domain of the HCN4 protein.
SEQ ID NO:2 represents the amino acid sequence of a portion of an extracellular domain of the HCN4 protein.
SEQ ID NO:3 represents the amino acid sequence of a peptide used for generating an HCN4 specific antibody.
SEQ ID NO:4 represents the amino acid sequence of a peptide used for generating an HCN4 specific antibody.
SEQ ID NO:5 represents the nucleotide sequence of a gene specific primer used in PCR.
SEQ ID NO:6 represents the nucleotide sequence of a gene specific primer used in PCR.
SEQ ID NO:7 represents the nucleotide sequence of a gene specific primer used in PCR.
SEQ ID NO:8 represents the nucleotide sequence of a gene specific primer used in PCR.
SEQ ID NO:9 represents the nucleotide sequence of a gene specific primer used in PCR.
SEQ ID NO:10 represents the nucleotide sequence of a gene specific primer used in PCR.
SEQ ID NO:11 represents the nucleotide sequence of a gene specific primer used in PCR.
SEQ ID NO:12 represents the nucleotide sequence of a gene specific primer used in PCR.
SEQ ID NO:13 represents the nucleotide sequence of a gene specific primer used in PCR.
SEQ ID NO:14 represents the nucleotide sequence of a gene specific primer used in PCR.
SEQ ID NO:15 represents the nucleotide sequence of a gene specific primer used in PCR.
SEQ ID NO:16 represents the nucleotide sequence of a gene specific primer used in PCR.
SEQ ID NO:17 represents the nucleotide sequence of a gene specific primer used in PCR.
SEQ ID NO:18 represents the nucleotide sequence of a gene specific primer used in PCR.
SEQ ID NO:19 represents the nucleotide sequence of a gene specific primer used in PCR.
SEQ ID NO:20 represents the nucleotide sequence of a gene specific primer used in PCR.
SEQ ID NO:21 represents the nucleotide sequence of a gene specific primer used in PCR.
SEQ ID NO:22 represents the nucleotide sequence of a gene specific primer used in PCR.
SEQ ID NO:23 represents the nucleotide sequence of a gene specific primer used in PCR.
SEQ ID NO:24 represents the nucleotide sequence of a gene specific primer used in PCR.
SEQ ID NO:25 represents the nucleotide sequence of a gene specific primer used in PCR.
SEQ ID NO:26 represents the nucleotide sequence of a gene specific primer used in PCR.
SEQ ID NO:27 represents the nucleotide sequence of a gene specific primer used in PCR.
SEQ ID NO:28 represents the nucleotide sequence of a gene specific primer used in PCR.
SEQ ID NO:29 represents the nucleotide sequence of a gene specific primer used in PCR.
SEQ ID NO:30 represents the nucleotide sequence of a gene specific primer used in PCR.
SEQ ID NO:31 represents the nucleotide sequence of a gene specific primer used in PCR.
SEQ ID NO:32 represents the nucleotide sequence of a gene specific primer used in PCR.
SEQ ID NO:33 represents the nucleotide sequence of a gene specific primer used in PCR.
SEQ ID NO:34 represents the nucleotide sequence of a gene specific primer used in PCR.
SEQ ID NO:35 represents the nucleotide sequence of a gene specific primer used in PCR.
SEQ ID NO:36 represents the nucleotide sequence of a gene specific primer used in PCR.
SEQ ID NO:37 represents the nucleotide sequence of a gene specific primer used in PCR.
SEQ ID NO:38 represents the nucleotide sequence of a gene specific primer used in PCR.
SEQ ID NO:39 represents the nucleotide sequence of a gene specific primer used in PCR.
SEQ ID NO:40 represents the nucleotide sequence of a gene specific primer used in PCR.
SEQ ID NO:41 represents the nucleotide sequence of a gene specific primer used in PCR.
SEQ ID NO:42 represents the nucleotide sequence of a gene specific primer used in PCR.
SEQ ID NO:43 represents the nucleotide sequence of a gene specific primer used in PCR.
SEQ ID NO:44 represents the nucleotide sequence of a gene specific primer used in PCR.
SEQ ID NO:45 represents the nucleotide sequence of a gene specific primer used in PCR.
SEQ ID NO:46 represents the nucleotide sequence of a gene specific primer used in PCR.

### [Sequence Listing]

C:¥Documents and Settings¥common¥desktop¥669871SEQUENCE LISTING.txt

## Claims

1. A method for preparing an implant which acts as a cardiac pacemaker, comprising the following steps:
(i) preparing cell aggregates, each of which comprises 3,000 cells of pacemaker cells derived from embryonic stem (ES) cells or induced pluripotent stem (iPS) cells; and
(ii) gathering the cell aggregates to form a gathered cell aggregate as an implant which comprises 60,000 or more pacemaker cells,
wherein the pacemaker cells possess HCN4 channel and Na channel, the beating rate of which can be controlled by regulation of Na channel.

2. The method according to claim 1, wherein the pacemaker cells are obtained by inducing differentiation of embryonic stem (ES) cells or induced pluripotent stem (iPS) cells into cardiac muscle cells, and selecting for the pacemaker cells using HCN4 channel as a marker.

3. The method according to claim 1, wherein the pacemaker cells are obtained by introducing an HCN4 channel gene into embryonic stem (ES) cells or induced pluripotent stem (iPS) cells, subjecting to differentiation induction into cardiac muscle cells, and screening cells exhibiting expression of the HCN4 channel gene.

4. The method according to claim 3, wherein the pacemaker cells are obtained by incorporating a gene encoding a screenable label into a HCN4 channel gene to construct a targeting gene, introducing the targeting gene into embryonic stem (ES) cells or induced pluripotent stem (iPS) cells, and screening cells positive for the label.

5. An implant acting as a cardiac pacemaker, comprising 60,000 or more embryonic stem (ES) cell or induced pluripotent stem (iPS) cell derived pacemaker cells, wherein:
the implant is a gathered cell aggregate comprising a plurality of cell aggregates, each cell aggregate having 3,000 cells of pacemaker cells;
the pacemaker cells possess HCN4 channel and Na channel; and
the implant is capable of controlling the beating rate by control of Na channel.

6. An implant according to claim 5, wherein the pacemaker cells are derived from embryonic stem (ES) cells or induced pluripotent stem (iPS) cells into which an HCN4 channel gene has been introduced.

7. An implant according to claim 6, wherein the HCN4 channel gene incorporates a gene encoding a screenable label.

8. A cardiac pacemaker comprising, as the essential component, an implant according to any one of claims 5 to 7, and is capable of controlling the beating rate by control of Na channel.

9. Use of an implant according to any one of claims 5 to 7 for the production of a cardiac pacemaker capable of controlling the beating rate by control of Na channel.

10. A method for the producing a cardiac pacemaker capable of controlling the beating rate by control of Na channel, comprising using an implant according to any one of claims 5 to 7.

11. The method according to any one of claims 2 to 4, comprising selecting or screening for the pacemaker cells using an HCN4 specific antibody which is obtained by using an amino acid sequence VSINGMVNNSW (SEQ ID NO: 1) or VPMLQDFPHD (SEQ ID N0:2) as the antigen and recognizes said amino acid sequence as the recognition site.

## Patentansprüche

1. Verfahren zur Herstellung eines Implantats, das als Herzschrittmacher fungiert, wobei das Verfahren folgende Schritte umfasst:
(i) das Herstellen von Zellaggregaten, die jeweils 3.000 Schrittmacherzellen umfassen, die von embryonalen Stammzellen (ES-Zellen) oder induzierten pluripotenten Stammzellen (iPS-Zellen) abgeleitet sind, und
(ii) das Zusammenfassen der Zellaggregate zur Ausbildung eines zusammengefassten Zellaggregats als Implantat, das 60.000 oder mehr Schrittmacherzellen umfasst,
wobei die Schrittmacherzellen einen HCN4-Kanal und einen Na-Kanal besitzen und ihre Schlagfrequenz durch Regulierung des Na-Kanals gesteuert werden kann.

2. Verfahren nach Anspruch 1, wobei die Schrittmacherzellen durch Induzieren der Differenzierung von embryonalen Stammzellen (ES-Zellen) oder induzierten pluripotenten Stammzellen (iPS-Zellen) zu Herzmuskelzellen und Selektion der Schrittmacherzellen unter Verwendung des HCN4-Kanals als Marker erhalten werden.

3. Verfahren nach Anspruch 1, wobei die Schrittmacherzellen durch Einführung eines HCN4-Kanal-Gens in embryonale Stammzellen (ES-Zellen) oder induzierte pluripotente Stammzellen (iPS-Zellen), Unterziehen derselben einer Differenzierungsinduktion zu Herzmuskelzellen und Screenen auf Zellen, die das HCN4-Kanalgen exprimieren, erhalten werden.

4. Verfahren nach Anspruch 3, wobei die Schrittmacherzellen durch Inkorporieren eines Gens, das für eine screenbare Markierung kodiert, in ein HCN4-Kanal-Gen zur Konstruktion eines Targeting-Gens, Einführen des Targeting-Gens in embryonale Stammzellen (ES-Zellen) oder induzierte pluripotente Stammzellen (iPS-Zellen) und Screening auf bezüglich der Markierung positive Zellen erhalten werden.

5. Implantat, das als Herzschrittmacher fungiert und 60.000 oder mehr von embryonalen Stammzellen (ES-Zellen) oder pluripotenten Stammzellen (iPS-Zellen) abgeleiteten Schrittmacherzellen umfasst, wobei:
das Implantat ein zusammengefasstes Zellaggregat ist, das eine Vielzahl von Zellaggregaten umfasst, die jeweils 3.000 Zellen als Schrittmacherzellen aufweisen;
die Schrittmacherzellen einen HCN4-Kanal und einen Na-Kanal besitzen; und
das Implantat in der Lage ist, durch Steuerung des Na-Kanals die Schlagfrequenz zu steuern.

6. Implantat nach Anspruch 5, wobei die Schrittmacherzellen von embryonalen Stammzellen (ES-Zellen) oder induzierten pluripotenten Stammzellen (iPS-Zellen) abgeleitet sind, in die ein HCN4-Kanal-Gen eingeführt wurde.

7. Implantat nach Anspruch 6, wobei das HCN4-Kanal-Gen ein inkorporiertes Gen umfasst, das für eine screenbare Markierung kodiert.

8. Herzschrittmacher, der als wesentliche Komponente ein Implantat nach einem der Ansprüche 5 bis 7 umfasst und in der Lage ist, durch Steuerung des Na-Kanals die Schlagfrequenz zu steuern.

9. Verwendung eines Implantats nach einem der Ansprüche 5 bis 7 zur Herstellung eines Herzschrittmachers, der in der Lage ist, durch Steuerung des Na-Kanals die Schlagfrequenz zu steuern.

10. Verfahren zur Herstellung eines Herzschrittmachers, der in der Lage ist, durch Steuerung des Na-Kanals die Schlagfrequenz zu steuern, wobei das Verfahren die Verwendung eines Implantats nach einem der Ansprüche 5 bis 7 umfasst.

11. Verfahren nach einem der Ansprüche 2 bis 4, umfassend das Selektieren oder Screenen auf die Schrittmacherzellen unter Verwendung eines HCN4-spezifischen Antikörpers, der unter Verwendung der Aminosäuresequenz VSINGMVNNSW (Seq.-ID Nr. 1) oder VPMLQDFPHD (Seq.-ID Nr. 2) als Antigen erhalten wird und die Aminosäuresequenz als Erkennungsstelle erkennt.

## Revendications

1. Procédé pour préparer un implant qui agit comme un simulateur cardiaque, comprenant les étapes suivantes consistant à :
(i) préparer des agrégats de cellules, chacun d'entre eux comprenant 3 000 cellules de cellules de stimulateur cardiaque dérivées de cellules souches embryonnaires (ES) ou de cellules souches pluripotentes induites (iPS) ; et
(ii) rassembler les agrégats de cellules pour former un agrégat de cellules rassemblées en tant qu'implant qui comprend 60 000 cellules de stimulateur cardiaque ou plus,
dans lequel les cellules de stimulateur cardiaque possèdent un canal HCN4 et un canal Na, dont le taux de battement peut être commandé par régulation du canal Na.

2. Procédé selon la revendication 1, dans lequel les cellules de stimulateur cardiaque sont obtenues en induisant une différenciation de cellules souches embryonnaires (ES) ou de cellules souches pluripotentes induites (iPS) dans des cellules de muscle cardiaque, et choisies pour les cellules de stimulateur cardiaque en utilisant un canal HCN4 en tant que marqueur.

3. Procédé selon la revendication 1, les cellules de stimulateur cardiaque sont obtenues par introduction d'un gène de canal HCN4 dans des cellules souches embryonnaires (ES) ou de cellules souches pluripotentes induites (iPS), soumission à une induction de différenciation dans des cellules de muscle cardiaque, et criblage de cellules présentant une expression du gène de canal HCN4.

4. Procédé selon la revendication 3, dans lequel les cellules de stimulateur cardiaque sont obtenues par incorporation d'un gène codant pour une étiquette pouvant être criblée dans un gène de canal HCN4 pour construire un gène ciblant, introduction du gène ciblant dans des cellules souches embryonnaires (ES) ou des cellules souches pluripotentes induites (iPS), et criblage de cellules positives pour l'étiquette.

5. Implant agissant comme un stimulateur cardiaque, comprenant 60 000 cellules de stimulateur cardiaque ou plus dérivées des cellules souches embryonnaires (ES) ou cellules souches pluripotentes induites (iPS), dans lequel :
l'implant est un agrégat de cellules rassemblées comprenant une pluralité de cellules agrégées, chaque agrégat de cellules ayant 3 000 cellules de cellules de stimulateur cardiaque ;
les cellules de stimulateur cardiaque possèdent un canal HCN4 et un canal Na ; et
l'implant est capable de commander le taux de battement en commandant le canal Na.

6. Implant selon la revendication 5, dans lequel les cellules de stimulateur cardiaque sont dérivées de cellules souches embryonnaires (ES) ou cellules souches pluripotentes induites (iPS) dans lesquelles un gène de canal HCN4 a été introduit.

7. Implant selon la revendication 6, dans lequel le gène de canal HCN4 incorpore un gène codant pour une étiquette pouvant être criblée.

8. Stimulateur cardiaque comprenant, comme composant essentiel, un implant selon l'une quelconque des revendications 5 à 7, et capable de commander le taux de battement en commandant le canal Na.

9. Utilisation d'un implant selon l'une quelconque des revendications 5 à 7 pour la production d'un stimulateur cardiaque capable de commander le taux de battement en commandant le canal Na.

10. Procédé pour la production d'un stimulateur cardiaque capable de commander le taux de battement en commandant le canal Na, comprenant l'utilisation d'un implant selon l'une quelconque des revendications 5 à 7.

11. Procédé selon l'une quelconque des revendications 2 à 4, comprenant la sélection ou le criblage des cellules de stimulateur cardiaque en utilisant un anticorps spécifique à HCN4 qui est obtenu en utilisant une séquence d'acides aminés VSINGMVNNSW (SEQ ID NO : 1) ou VPMLQDFPHD (SEQ ID NO : 2) en tant qu'antigène, et reconnaissant ladite séquence d'acides aminés comme le site de reconnaissance.
